# EUROPÄISCHE PATENTSCHRIFT

(11) **EP 1 865 862 B1**
(45) Veröffentlichungstag und Bekanntmachung des Hinweises auf die Patenterteilung: **24.08.2016**
(21) Anmeldenummer: 06724014.3
(22) Anmeldetag: 04.04.2006
(51) Int. Cl.: A61B 17/70, A61B 17/86

(54) **PEDIKELSCHRAUBE**
PEDICLE SCREW
VIS PÉDICULAIRE

(30) Priorität: 04.04.2005 EP 05007292
(43) Veröffentlichungstag der Anmeldung: 19.12.2007
(73) Patentinhaber: Zimmer GmbH, 8404 Winterthur (CH)
(72) Erfinder: CASUTT, Simon, CH-9200 Gossau (CH)
(74) Vertreter: Manitz, Finsterwald & Partner GbR
(86) Internationale Anmeldenummer: PCT/EP2006/003059
(87) Internationale Veröffentlichungsnummer: WO 2006/105935

(56) Entgegenhaltungen:
- EP-A- 0 669 109
- EP-A- 1 273 269
- WO-A-01/54598
- WO-A-2005/041793
- WO-A-2005/065374
- US-A1- 2005 055 025
- US-B1- 6 206 882
- US-B1- 6 656 184

## Beschreibung

Die Erfindung betrifft eine Pedikelschraube, umfassend einen Kopf zur Koppelung mit einem elastischen intervertebralen Stabilisierungs- oder Stützsystem, und einen zur Verankerung in einem Wirbel dienenden Schaft, der sich im implantierten Zustand durch den Pedikel hindurch in den Wirbelkörper erstreckt. Die Erfindung betrifft außerdem ein intervertebrales Stabilisierungssystem mit mehreren Pedikelschrauben.

Die Pedikelschrauben werden nachstehend auch als "Befestigungselemente" bezeichnet.

Pedikelschrauben dieser Art und intervertebrale Stabilisierungs- und Stützsysteme sind beispielsweise aus US 2005/0154390, US 5,492,442, WO2005/065374, WO03/032862, EP 0 669 109 A1, EP 0 672 388, US 4,950,269 oder EP 528 706 bekannt. Derartige Schrauben weisen als Kopf eine geschlossene oder einseitig geöffnete Öse auf, wobei letztere Form in bestimmten Ausführungsformen Tulpen- oder Stimmgabelform aufweist und die Öse vorgesehen ist, um die intervertebralen Stabilisierungs- oder Stützelemente aufzunehmen und durch geeignete Maßnahmen zu fixieren.

Die vorstehend genannte EP 0 669 109 A1 beschreibt ein elastisches Stabilisierungssystem sowie dafür vorgesehene Pedikelschrauben der vorstehend genannten Art und wie im Oberbegriff des Anspruchs 1 angegeben. Die bekannten Pedikelschrauben besitzen folglich einen Schaft zur Verankerung in einem Wirbel und einen als Öse ausgebildeten Kopf, der ein Band des Stabilisierungssystems aufnehmen kann.

Ein Pedikel, in den eine Schraube eingebracht werden soll, kann in einem vereinfachten mechanischen Modell als ein am Wirbelkörper befestigter Balken betrachtet werden, der sich bei Belastung signifikant durchbiegen kann. Dabei ist das Steifigkeitsprofil in Richtung der Längsachse des Pedikels nicht konstant. Vielmehr ist die Steifigkeit im Bereich des Übergangs zwischen Pedikel und Wirbelkörper im Vergleich zu den angrenzenden Bereichen deutlich erhöht. Dabei haben Untersuchungen ergeben, dass sich bei implantierten starren Pedikelschrauben, wie sie herkömmlicherweise verwendet werden, der Pedikel einschließlich der Schraube durchbiegt, wenn eine kaudal-kraniale Kraft am Schraubenkopf angreift. Aufgrund der deutlich höheren Biegesteifigkeit der Schraube bildet sich ein auch als Togglepoint bezeichneter Drehpunkt aus, um den die Pedikelschraube unter Belastung kippt. Dieser Drehpunkt liegt im Allgemeinen in etwa mehr oder weniger im Bereich des Übergangs zwischen Pedikel und Wirbelkörper. Das Verkippen der belasteten Pedikelschraube führt zu einer hohen Belastung der Spongiosa des Wirbelkörpers durch den freien Endbereich und insbesondere durch die Spitze der Pedikelschraube. Hierdurch kommt es zu starken Belastungen des so genannten Knocheninterface, was unter ungünstigen Umständen, beispielsweise bei fortschreitender Osteoporose, auf lange Sicht in Schraubenlockerungen resultieren kann. Außerdem werden hohe Spannungen innerhalb des Schaftes vor allem leicht anterior des Drehpunktes erzeugt.

Pedikelschrauben werden sowohl in Verbindung mit starren als auch elastischen intervertebralen Stabilisierungs- oder Fixationssystemen (Versteifungs- oder Stützsystemen) eingesetzt. Bei Systemen, welche eine nicht winkelstabile Verbindung oder Koppelung zwischen den intervertebralen Stützelementen, zum Beispiel zwischen einer an einem kaudalen und einer an einem kranialen Wirbel befestigten Pedikelschraube angeordneten Stäben oder Bändern, einerseits und den Pedikelschrauben andererseits vorsehen, wie beispielsweise elastischen, dynamisch stabilisierenden Systemen, werden im Wesentlichen keine Momente, sondern praktisch ausschließlich reine Zug-/Druckkräfte in den Schraubenkopf eingeleitet beziehungsweise dort aufgenommen. Dies hat zur Folge, dass der Pedikel sich durchbiegen kann. Die steife Schaube erfährt also eine Art Kippbeanspruchung, welche aufgrund der vergleichsweise geringen Biegesteifigkeit der Pedikel von diesen nicht abgestützt werden kann und daher im Bereich der Schraubenspitze am so genannten Knocheninterface hohe Kräfte in die Spongiosa des Wirbelkörpers einbringt.

Dieses Phänomen tritt im Übrigen bei Versteifungssystemen, welche eine winkelinvariante Kopplung beinhalten, in weitaus geringeren Maße auf als bei Systemen mit winkelvarianten Systemen: Aufgrund der Winkelinvariabilität wird eine Kippbelastung der Schraube verunmöglicht, d. h. das Moment, das bei einem winkelvarianten System die Kraft an der Schraubenspitze über den Hebelarm vom Togglepoint zur Schraubenspitze induziert, wird bei einem winkelinvarianten System direkt im Schaubenkopf ausgeglichen, wobei die nachfolgend angegebene und beanspruchte Pedikelschraube ohne weiteres auch für diese winkelinvarianten Systeme geeignet ist.

Aus der US 2005/154390 ist eine Pedikelschraube bekannt, welche in einem mehr oder weniger unmittelbar am Kopf angrenzenden und nach der Implantation außerhalb des Knochens liegenden Bereich eine biegeelastische Zone aufweist.

Es soll eine Pedikelschraube der eingangs genannten Art angegeben werden, welche neben anderen vorteilhaften Eigenschaften auch in der Lage ist, eine Lockerung der Schrauben zu vermeiden und gleichzeitig eine geeignete Abstützung von Momenten, welche durch den Angriff von Kräften am Schraubenkopf entstehen, zu gewährleisten.

Die in Anspruch 1 angegebene Pedikelschraube vermag, neben einer Vielzahl weiterer vorteilhafter Eigenschaften, diese Forderung zu erfüllen. Bei der erfindungsgemäßen Pedikelschraube ist eine Entlastungszone vorgesehen, die zwischen einem am Kopf angrenzenden oberen Schaftbereich und einem unteren Schaftbereich gelegen ist, wobei der obere Schaftbereich und der untere Schaftbereich eine höhere Biegesteifigkeit aufweisen als die Entlastungszone.

Die Pedikelschraube eignet sich unter anderem zur Verwendung in intervertebralen Versteifungs- oder Stützsystemen, welche eine nicht winkelstabile Verbindung zwischen den intervertebralen Stützelementen, zum Beispiel zwischen einer an einem kaudalen und einer an einem kranialen Wirbel befestigten Pedikelschraube angeordneten Stäben oder Bändern, und den Pedikelschrauben vorsehen. Ein System dieser Art wird ebenfalls angegeben und beansprucht. Selbstverständlich kann die hier angegebene Schraube auch in anderen Stütz- oder Versteifungssystemen oder sonstigen Anwendungen höchst vorteilhafte Wirkungen entfalten.

Bei der hier angegebenen Pedikelschraube ist der Schaft mit einer Entlastungszone versehen, die eine reduzierte Biegesteifigkeit gegenüber einem in Richtung des Kopfes angrenzenden oberen Schaftbereich aufweist, wobei die Entlastungszone im zur Anordnung im Knochen vorgesehenen Bereich des Schaftes gelegen ist.

Der Schaft des Befestigungselementes (Pedikelschraube) ist mit einer Entlastungszone versehen, die auch als flexible Übergangszone bezeichnet werden kann. Dies bedeutet eine Abkehr von herkömmlichen Pedikelschrauben, die über ihre gesamte Länge starr ausgebildet sind und eine im Vergleich zum Knochenmaterial der Wirbel hohe Biegesteifigkeit aufweisen.

Durch die Entlastungszone erhält der Schaft des Befestigungselementes ein Biegesteifigkeitsprofil, dessen Verlauf durch die Ausgestaltung der Entlastungszone prinzipiell beliebig eingestellt und insbesondere an die charakteristischen Eigenschaften des Wirbels angepasst werden kann. Es hat sich herausgestellt, dass durch die Entlastungszone die Übertragung von Biegemomenten, die durch am Kopf des Befestigungselementes angreifende Kräften verursacht werden, in den freien Endbereich des Befestigungselementes begrenzt werden kann. Der Schaft des Befestigungselementes kann sich unter Last aufgrund der Entlastungszone gewissermaßen im Gleichklang mit der Knochenstruktur, in die das Befestigungselement implantiert ist, durchbiegen. Hohe Belastungen des Knochenmaterials und hohe Spannungen im Schaft werden hierdurch vermieden. Die Anpassung der Biegesteifigkeit der Pedikelschraube an die biologischen Gegebenheiten schafft damit eine weiter verbesserte Sicherheit für den Patienten.

Bei dem weiterhin angegebenen und beanspruchten intervertebralen Stabilisierungssystem sind eine Mehrzahl von an den Wirbeln verankerbaren Pedikelschrauben und eine Verbindungseinrichtung zum Verbinden von zumindest zwei an benachbarten Wirbeln verankerten Pedikelschrauben zu einem elastischen Versteifungs- oder Stützsystem vorgesehen. Herkömmliche Pedikelschrauben besitzen häufig eine konische Form und damit streng genommen keine über die Schaftlänge konstante Biegesteifigkeit. Ein solches "Biegesteifigkeitsprofil" besitzt aber gerade keine Entlastungszone in dem hier angegebenen Sinne. Die hier angegebene Pedikelschraube basiert vielmehr auf dem Gedanken, die Biegesteifigkeit des Schaftes in einem bestimmten axialen Bereich gezielt herabzusetzen.

Pedikelschrauben werden üblicherweise im Bereich der Lendenwirbel L1 bis L5 implantiert. Obwohl diese Wirbel weder bei demselben Patienten noch bei unterschiedlichen Patienten identisch ausgebildet sind, stimmen alle für die Implantation von Pedikelschrauben in Frage kommenden Wirbel hinsichtlich bestimmter Charakteristiken überein, wie vorstehend bereits erwähnt. Dieser Umstand, auf den nachstehend näher eingegangen wird, kann bei der hier angegebenen Pedikelschraube genutzt werden, indem das Biegesteifigkeitsprofil des Schaftes gezielt an die von Pedikel und Wirbelkörper gebildete Knochenstruktur angepasst wird. Die hier angegebene Pedikelschraube ist aber nicht auf die genannten Lendenwirbel beschränkt. Insbesondere ist grundsätzlich auch eine Anwendung für die thorokalen Wirbel möglich.

Weitere Ausführungsformen sind auch in den abhängigen Ansprüchen, der Beschreibung sowie der Zeichnung angegeben, wobei die Merkmale dieser Ausführungsformen in an sich beliebiger Weise miteinander kombinierbar sind.

In einer Ausführungsform ist die Biegesteifigkeit der Entlastungszone im Wesentlichen der eines Pedikels angepasst oder liegt leicht darüber. Die Biegesteifigkeit beträgt beispielsweise das 1,5- bis 4-fache, das 1,5- bis 3-fache, das 2- bis 4-fache oder das 2- bis 3-fache der Biegesteifigkeit der Pedikel, wobei dieser Wert abhängig von der gesamten Geometrie der Schraube so zu wählen ist, dass insbesondere im Bereich der Schraubenspitze sich eine Flächenpressung des Spongiosagewebes einstellt, die unter der maximal zulässigen Flächenpressung liegt, dabei aber unter Berücksichtigung eines Sicherheitsfaktors eine möglichst gute Ausnutzung der zulässigen Belastung erlaubt.

Der Kopf kann mit dem oberen Schaftbereich wenigstens in Biegerichtung des Schaftes starr gekoppelt und insbesondere einteilig sein. Im Falle einer nicht einteiligen Ausführung ist es dabei möglich, aber nicht zwingend, dass die Koppelung zwar bezüglich einer Biegung fest, eine Torsion dagegen aber möglich ist.

Der Gradient der Biegesteifigkeit am Übergang vom oberen Schaftbereich zur Entlastungszone kann betragsmäßig größer, insbesondere signifikant größer, sein als ein im oberen Schaftbereich auftretender Biegesteifigkeitsgradient.

Am Übergang vom oberen Schaftbereich zur Entlastungszone kann wenigstens in einem Bereich des Schaftes der Betrag des Gradienten der Biegesteifigkeit wenigstens doppelt so groß, insbesondere wenigstens 5mal so groß, und weiterhin insbesondere wenigstens 10mal so groß sein wie im oberen Schaftbereich.

Die Biegesteifigkeit kann am Übergang im Wesentlichen sprunghaft reduziert sein.

Die Biegesteifigkeit in der Entlastungszone kann in wenigstens einer Biegeebene gegenüber der Biegesteifigkeit im oberen Schaftbereich um wenigstens 30%, insbesondere um wenigstens 50%, in einer weiteren Ausführungsform um wenigstens 60%, und in einer noch weiteren Ausführungsform um wenigstens 80% geringer sein.

Der obere Schaftbereich kann derart dimensioniert sein, dass die Entlastungszone im zur Anordnung in der Spongiosa vorgesehenen Bereich des Schaftes gelegen ist, während wenigstens der zur Anordnung in der Kortikalis vorgesehene Bereich von dem oberen Schaftbereich gebildet ist. Der obere Schaftbereich dient somit zur Abstützung am festen Knochenmaterial.

Die Länge des oberen Schaftbereiches kann im Minimum 5mm, insbesondere im Minimum 8mm sowie im Maximum 15mm, und insbesondere im Maximum 12mm betragen.

Wie vorstehend bereits angedeutet, besitzt die Biegesteifigkeit bzw. die maximal zulässige Flächenpressung im Wirbel einen für alle Wirbel prinzipiell gleichen charakteristischen Verlauf, bezogen auf eine durch den Pedikel und den Wirbelkörper verlaufende Achse, die mit der Mittelachse des Befestigungselementes im implantierten Zustand zusammenfällt.

Dieser Umstand kann ausgenutzt werden, indem ein axiales Biegesteifigkeitsprofil des Schaftes wenigstens an den qualitativen Verlauf der Biegesteifigkeit des Pedikels angenähert ist und diesem im Idealfall entspricht. Hierdurch kann erreicht werden, dass die von der Pedikelschraube ausgeübte, senkrecht zu ihrer Längserstreckung verlaufende Querkraft entlang ihrer Längserstreckung zumindest näherungsweise konstant ist.

Am Übergang von der Entlastungszone zum unteren Schaftbereich kann wenigstens in einem Bereich des Schaftes der Betrag des Gradienten der Biegesteifigkeit wenigstens doppelt so groß, insbesondere wenigstens 5mal so groß, und weiterhin insbesondere wenigstens 10mal so groß sein wie im unteren Schaftbereich.

Bei dieser Ausgestaltung ist also auch der - vom Kopf aus gesehen - hinter der Entlastungszone gelegene untere Schaftbereich biegesteifer als die Entlastungszone. Diese Ausgestaltung des Befestigungselementes ist aber nicht zwingend. Prinzipiell ist es vielmehr auch möglich, eine sich bis zum freien Ende des Befestigungselementes erstreckende Entlastungszone vorzusehen. Bei entsprechender Ausgestaltung insbesondere der Spitze des Befestigungselementes kann hierdurch ein "Führungseffekt" im Pedikel genutzt werden. Das Befestigungselement wird beim Einbringen gewissermaßen "automatisch" von der relativ harten kortikalen Außenschicht korrigierend abgelenkt; mit einer entsprechend ausgebildeten Pedikelschraube könnte somit gewissermaßen "um die Ecke" geschraubt werden.

Der Wechsel der Biegesteifigkeit von der Entlastungszone zum unteren Schaftbereich kann im Wesentlichen sprunghaft erfolgen.

Das Profil des Biegesteifigkeitsverlaufs zwischen dem oberen Schaftbereich und dem unteren Schaftbereich kann im Wesentlichen topf-, trog- oder wannenförmig ausgebildet sein.

Die Lage sowie die axiale Länge der Entlastungszone im Schaft sind auf die Lage der Übergangszone zwischen Pedikel und Wirbelkörper im Wirbel abgestimmt, für den das Befestigungselement konzipiert ist. Da die Knochenstruktur von Wirbeln hinreichend bekannt ist, steht hinreichend genau fest, an welcher Stelle längs des implantierten Schaftes die erwähnte Übergangszone sowie der vorstehend bereits erwähnte Togglepoint liegen. Dies gilt zumindest jeweils bezogen auf eine bestimmte, vom Operateur gewählte Setztechnik. So ist beispielsweise zu berücksichtigen, dass z.B. für eine so genannte bikortikale Verankerung vergleichsweise lange Pedikelschrauben zum Einsatz kommen. Vor diesem Hintergrund kann bei der hier angegebenen Pedikelschraube die Entlastungszone in einem mittleren Bereich der Längserstreckung des Schaftes gelegen sein, insbesondere im Wesentlichen in den beiden mittleren Vierteln des Schaftes oder im mittleren Drittel des Schaftes.

Die Entlastungszone kann eine signifikante axiale Länge des Schaftes einnehmen. Wenn der Schaft mit einem Gewinde versehen ist, dann kann sich die Entlastungszone in Längsrichtung über eine Mehrzahl von Gewindegängen erstrecken.

Der obere Schaftbereich und die Entlastungszone können derart dimensioniert sein, dass im implantierten Zustand sich die Entlastungszone im Bereich des Übergangs zwischen Pedikel und Wirbelkörper befindet und insbesondere sich auf beiden Seiten axial über den Übergangsbereich hinaus erstreckt, wobei in einer Ausführungsform vom Kopf aus gesehen ein hinter dem Übergang gelegener Abschnitt der Entlastungszone eine größere axiale Länge aufweist als ein vor dem Übergang gelegener Abschnitt der Entlastungszone.

Der Schaft kann mit einem Gewinde versehen sein, wobei das Gewinde durch die Entlastungszone unterbrochen ist.

Der Schaft kann zumindest bereichsweise hohl ausgeführt und insbesondere mit einer zentralen Längsbohrung versehen sein.

Die Längsbohrung kann durchgehend ausgeführt sein, wobei dies allerdings nicht zwingend ist. Eine durchgehende Bohrung hat unter anderem den Vorteil, dass das Befestigungselement bei der Implantation beispielsweise mittels eines Kirschnerdrahtes geführt werden kann.

Eine hohle Ausgestaltung des Schaftes ist allerdings nicht zwingend. Der Schaft kann auch massiv ausgeführt sein, wobei eine massive Ausführung des Schaftes auch in der Entlastungszone vorgesehen sein kann.

Der Schaft kann zumindest in der Entlastungszone mit einer Querschnittsschwächung im Vergleich zum oberen Schaftbereich und insbesondere auch zu einem unteren Schaftbereich versehen sein.

Die reduzierte Biegesteifigkeit in der Entlastungszone des Schaftes kann dadurch realisiert werden, dass der Schaft in der Entlastungszone mit einer Schwächung durch Materialwegnahme versehen ist. Diese Materialwegnahme kann derart erfolgen, dass das Flächenmoment des Schaftes unter gleichzeitiger optimaler Ausnutzung des den Schaft bildenden Werkstoffes reduziert ist.

Für die Schwächung durch Materialwegnahme gibt es zahlreiche in der Praxis realisierbare Möglichkeiten. Beispiele sind im Folgenden kurz skizziert. Auf entsprechende konkrete Ausgestaltungen wird in Verbindung mit der Beschreibung der Zeichnungen näher eingegangen.

Die Entlastungszone kann von einem lang gestreckten Schaftbereich mit im Vergleich zum oberen Schaftbereich und insbesondere auch zu einem unteren Schaftbereich reduzierter Querschnittsfläche gebildet sein.

Der Schaft kann zumindest in der Entlastungszone als Wendel ausgebildet sein.

Der Schaft kann zumindest in der Entlastungszone mit wenigstens einer nut- oder schlitzartigen Vertiefung versehen sein, die insbesondere wendelformig umläuft. Die umlaufende Vertiefung kann gleichsinnig oder gegensinnig in Bezug auf ein am Schaft ausgebildetes Gewinde orientiert sein.

Ist der Schaft zumindest bereichsweise hohl ausgeführt, bzw. mit einer zentralen Längsbohrung versehen, kann vorgesehen sein, dass in den hohl ausgeführten Schaftbereichen die Wandung des Schaftes durchbrochen ist.

Handelt es sich um einen mit einem Gewinde versehenen Schaft, so kann die Wandung im Gewindetal durchbrochen sein. Möglich ist aber auch eine Ausführung, bei der die Wandung in der Gewindespitze unterbrochen ist.

Eine derartige Ausgestaltung des Schaftes eignet sich auch für solche Befestigungselemente, die nicht in den Wirbel eingedreht, sondern vielmehr eingeschlagen werden, da aufgrund der geringen Schlitzbreite die Einschlagimpulse problemlos in axialer Richtung übertragen werden können.

Bei dem hier angegebenen Befestigungselement muss es sich nicht zwingend um Schrauben im herkömmlichen Sinne, sondern es kann sich auch um durch Einschlagen implantierbare Befestigungselemente handeln. Nichtsdestotrotz kann der Schaft eines solchen Einschlagelementes mit einem Gewindeansatz versehen sein, der das Einschlagen nicht behindert, ein Explantieren durch Herausdrehen aber erleichtert bzw. ermöglicht.

Die Wandung des Schaftes kann wenigstens in der Entlastungszone zwei wendelförmig umlaufende schlitzförmige Durchbrechungen aufweisen.

Der Schaft kann wenigstens im Bereich der Entlastungszone eine Doppelwendel ausbilden.

Die Steigung der Wendel im Bereich der Entlastungszone kann wenigstens 5mm, insbesondere wenigstens 10 mm, betragen.

Die Steigung der Wendel kann über die Längserstreckung des Schaftes variieren, wodurch die Biegesteifigkeit des Schaftes über die Längserstreckung variiert.

Die Herstellung einer Entlastungszone mit durchgehenden, auch wendelförmig oder doppelwendelförmig umlaufenden Nuten oder Schlitzen kann beispielsweise durch ein an sich aus dem Stand der Technik bekanntes Drahterosionsverfahren erfolgen. Drahterodierverfahren sind beispielsweise in DE 101 96 821 T5 beschrieben worden. Bei der hier beschriebenen

Anwendung wird eine Querbohrung in den Schaft eingebracht, durch welche der für das Erosionsverfahren verwendete Draht hindurchgeführt wird. Während des Erosionsvorgangs wird entweder die Schraube oder der Draht in Axialrichtung der Schraube vorgeschoben. Während der Vorschubbewegung wird die Schraube in einer Ausführungsform des Verfahrens um ihre Längsachse gedreht. Dadurch entsteht ein wendelförmiger Schlitz beziehungsweise es werden simultan zwei wendelförmig umlaufende Schlitze erzeugt. Ohne die Rotation der Schraube entsteht ein gerader Schlitz. Die Bearbeitung erfolgt insbesondere zur Einspannstelle der Schraube hin oder bei beidseitiger Einspannung zu der Einspannstelle hin, über welche die Vorschub- und/oder Drehkraft aufgebracht wird. Dies gewährleistet eine gute Kraftübertragung während des Bearbeitungsvorgangs. In einer Variante des Herstellverfahrens wird die Schraube am Kopf eingespannt bzw. angetrieben und der Erosionsvorgang erfolgt zum Kopf hin.

Der Schaft kann zumindest in der Entlastungszone mit senkrecht oder schräg zur Schaftachse verlaufenden Querbohrungen versehen sein.

Es können von der Außenwand des Schaftes zu den Querbohrungen führende Schlitze angeordnet sein, deren Breite kleiner ist als der Durchmesser der Querbohrungen.

Der Schaft kann mit mehreren in axialer Richtung hintereinander und insbesondere versetzt angeordneten, schlitz-, nut- oder kerbartigen Vertiefungen versehen sein, wobei in einer Ausführungsform die Tiefe der Vertiefungen jeweils größer ist als der im Bereich der Vertiefung gemessene halbe Durchmesser des Schaftes.

Es ist nicht erforderlich, dass das Biegesteifigkeitsprofil des Schaftes in allen die Mittelachse des Schaftes enthaltenen Ebenen identisch ist, d.h. eine Rotationssymmetrie des Biegesteifigkeitsprofils - bezogen auf die Mittelachse des Schaftes - ist nicht zwingend. Folglich kann das Biegesteifigkeitsprofil des Schaftes bezogen auf dessen Längsachse rotationsunsymmetrisch sein.

Die Entlastungszone kann derart ausgebildet sein, dass die Biegesteifigkeit in einer Ebene am geringsten ist, die von der Längsachse des Schaftes und der Richtung einer im implantierten Zustand über den Köpf aufgebrachten intervertebralen Kraft aufgespannt ist, wobei in einer Ausführungsform die Biegesteifigkeit in einer senkrecht dazu aufgespannten Ebene am größten ist.

Der Schaft im Bereich der Entlastungszone kann einen rotationsunsymmetrischen Querschnitt aufweisen, und insbesondere in der Ebene der größten Biegesteifigkeit im Wesentlichen eine identische Abmessung aufweisen wie die unmittelbar benachbarten oberen und unteren Schaftbereiche.

Durch eine solche Ausgestaltung kann erreicht werden, dass die Steifigkeit des Schaftes hinsichtlich Torsion sowie in Bezug auf Zug-/Druckbelastungen so wenig wie möglich beeinträchtigt wird. Bei einer Pedikelschraube wird durch diese Ausgestaltung das Eindrehverhalten so wenig wie möglich beeinträchtigt.

Der Schaft kann in der Entlastungszone wenigstens einen den Schaft durchdringenden und im Wesentlichen in Längsrichtung des Schaftes verlaufenden Schlitz aufweisen. Ein derartiger geschlitzter Bereich des Schaftes kann gewissermaßen als eine Wendel mit "unendlich großer" Wendelsteigung bezeichnet werden und stellt insofern einen Spezialfall einer wendelförmigen bzw. doppelwendelförmigen Entlastungszone dar, und kann ebenfalls wie oben beschrieben durch ein Drahterosionsverfahren ohne Drehung der Schraube hergestellt werden.

Der obere Schaftbereich kann wenigstens eine im Wesentlichen in Längsrichtung verlaufende Vertiefung an der Oberfläche aufweisen. In derartige Vertiefungen kann der Knochen einwachsen, wodurch eine Verdrehsicherung geschaffen wird.

Der Schaft kann einstückig ausgebildet ein.

Die Entlastungszone kann zumindest zum Teil von einem Zwischenstück aus einem vom übrigen Schaftmaterial abweichenden Material gebildet sein.

Das Zwischenstück kann aus einem insbesondere faserverstärkten Kunststoffmaterial, insbesondere einem Polymermaterial, hergestellt sein.

An die Entlastungszone angrenzende Schaftbereiche können durch ein in der Entlastungszone gelegenes Gelenk miteinander verbunden sein.

Die Entlastungszone kann durch ein Gelenk gebildet sein, das zwei unmittelbar aneinander angrenzende Schaftbereiche miteinander verbindet.

Bei den Ausführungsformen, die mit einem zumindest bereichsweise hohlen Schaft und in diesem Bereich mit zumindest einer wendelförmig umlaufenden Durchbrechung der Wandung versehen sind, kann die Herstellung der Durchbrechung derart erfolgen, dass beim Durchdringen der Gewindespitzen eines in diesem Bereich vorgesehenen Gewindes Schneiden erzeugt werden, die ein Eindrehen der Pedikelschraube erleichtern können.

Die Herstellung einer Pedikelschraube kann durch ein Drahterosionsverfahren erfolgen, bei dem ausgehend von einem zumindest bereichsweise hohlen Schaft ein sich durch den Schaft hindurch erstreckender Draht relativ zur Pedikelschraube derart geführt wird, dass in der Wandung zwei wendelförmig umlaufende schlitzförmige Durchbrechungen mit einer vorgegebenen Wendelsteigung entstehen.

Durchbiegungsversuche an auf diese Weise hergestellten Pedikelschrauben haben ergeben, dass ausgehend von herkömmlichen Pedikelschrauben, bei denen für eine Durchbiegung von 1 mm etwa eine Kraft von 150 N erforderlich ist, diese Kraft auf 15 N reduziert wird, wenn die Wendelsteigung etwa 9 mm beträgt. Weiter hat sich gezeigt, dass durch Verringern der Wendelsteigung erreicht werden kann, dass eine Kraft von 1 N eine Durchbiegung vom mehr als 1 mm erzielen kann. Es hat sich also gezeigt, dass sich die Biegesteifigkeit als eine Funktion primär der Wendelsteigung darstellen lässt.

Der Schaft des Befestigungselementes kann einen kreisförmigen Querschnitt aufweisen. Eine solche Kreiszylinderform ist aber nicht zwingend. Vielmehr ist es möglich, dass der Schaft einen von einer Kreisform abweichenden Querschnitt aufweist, beispielsweise einen elliptischen oder ovalen Querschnitt. Hierdurch kann dem Umstand Rechnung getragen werden, dass Pedikel keine Kreiszylinderform, sondern einen ovalen Querschnitt aufweisen. Ein Befestigungselement mit einem entsprechend geformten Schaft kann sich daher besser an der kortikalen Wand des Pedikels abstützen als ein Schaft mit Kreiszylinderform, wodurch eine verbesserte Verankerung im Pedikel erzeugt werden kann.

Der Schaft kann aus einem Memory-Metall hergestellt sein, beispielsweise aus einem Memory-Metall auf NiTi-Basis.

Intervertebrale Stabilisierungssysteme mit durch die Pedikel hindurch in den Wirbelkörper zu verankernden Befestigungselementen, wie insbesondere Pedikelschrauben, sind grundsätzlich bekannt. Wie eingangs bereits erläutert, kann man dabei grundsätzlich unterscheiden zwischen starren bzw. winkelstabilen Systemen bzw. Systemen mit winkelinvarianter Ankoppelung einerseits und dynamischen oder elastischen Systemen bzw. Systemen mit winkelvarianter Ankoppelung andererseits. Ein dynamisches intervertebrales Stabilisierungssystem beispielsweise wird von der Anmelderin unter der Produktbezeichnung "Dynesys" vertrieben und ist beispielsweise in der EP 669 109 beschrieben.

Gemeinsam ist allen derartigen Stabilisierungssystemen, dass deren Befestigungselemente, insbesondere Pedikelschrauben, in zumindest zwei benachbarten Wirbeln verankert und durch eine Verbindungseinrichtung miteinander verbunden sind. Für die Ausgestaltung dieser Verbindungseinrichtung existiert eine Vielzahl von Möglichkeiten. So kann die Verbindungseinrichtung beispielsweise einen starren oder elastischen Stab umfassen, mit dem wenigstens zwei Befestigungselemente bzw. Pedikelschrauben starr miteinander verbunden sind.

Zur Realisierung eines dynamischen oder elastischen Systems kann die Verbindungseinrichtung ein auf Zug vorspannbares Band umfassen, das im implantierten Zustand von wenigstens einem zwischen zwei benachbarten Pedikelschrauben angeordneten komprimierbaren Druckkörper umgeben ist ("Dynesys").

Grundsätzlich bei allen intervertebralen Stabilisierungssystemen können nun die hier angegebenen Pedikelschrauben vorgesehen werden. Da intervertebrale Stabilisierungssysteme an sich grundsätzlich bekannt sind, wird auf diese Systeme im Folgenden nicht näher eingegangen.

Die Erfindung wird nachstehend beispielhaft unter Bezugnahme auf die beigefügte Zeichnung beschrieben. Es zeigen:
- Fig. 1: eine Pedikelschraube gemäß dem Stand der Technik,
- Fig. 2: die Pedikelschraube von Fig. 1 zusammen mit einem Wirbel zur Erläuterung bestimmter Abmessungen,
- Fig. 3: ein Befestigungselement zusammen mit Kurven unterschiedlicher Biegesteifigkeitsprofile,
- Fig. 4-16: verschiedene Ausführungsformen von Befestigungselementen, und
- Fig. 17: eine Darstellung zur Erläuterung eines intervertebralen Stabilisierungssystems.

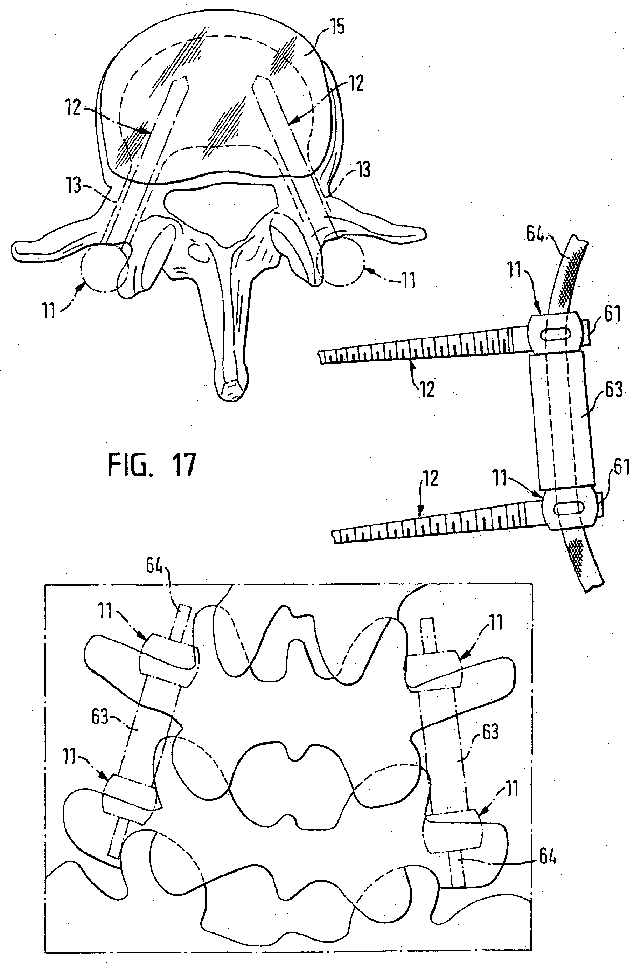

Die im Folgenden beschriebenen Befestigungselemente sind für ein dynamisches bzw. elastisches intervertebrales Stabilisierungssystem konzipiert, beispielsweise für das Dynesys-System der Anmelderin, wie es vorstehend bereits erläutert wurde (vgl. auch Fig. 17 mit zugehöriger Erläuterung am Ende der Beschreibung). Befestigungselemente wie Pedikelschrauben für intervertebrale Stabilisierungssysteme variieren vor allem im Bereich des Kopfes, da der Kopf zur Koppelung mit der Verbindungseinrichtung dient, durch die Befestigungselemente bzw. Pedikelschrauben benachbarter Wirbel miteinander verbunden sind. Die Köpfe der nachstehend beschriebenen Befestigungselemente sind für das Dynesys-System der Anmelderin ausgelegt. Die Auslegung des Schaftes der einzelnen Befestigungselemente kann prinzipiell mit beliebig ausgestalteten Köpfen kombiniert werden und ist daher grundsätzlich für beliebige intervertebrale Stabilisierungssysteme einsetzbar.

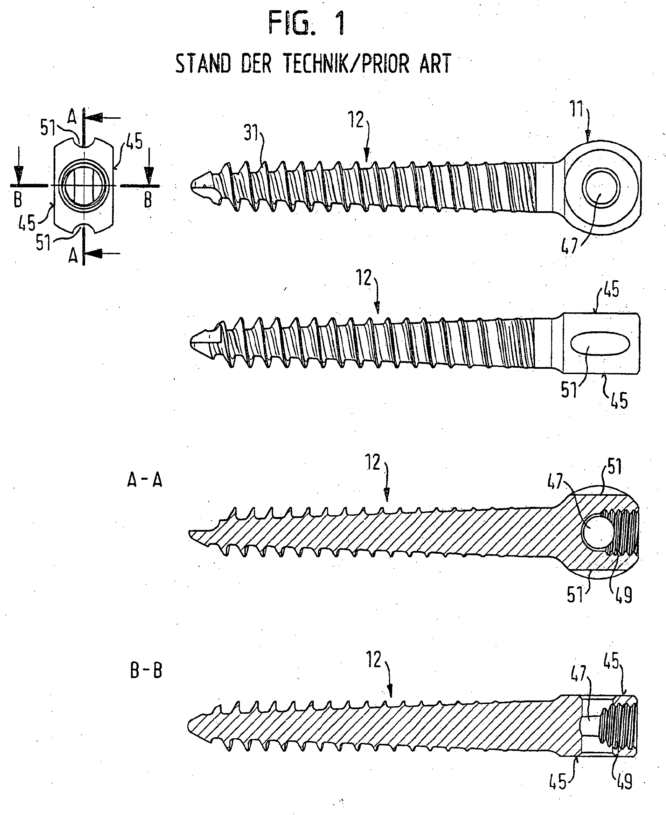

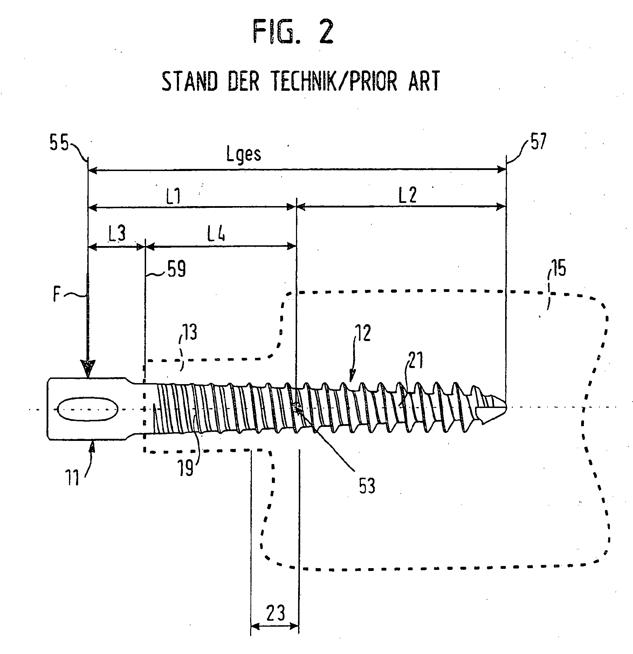

Die Fig. 1 und 2 zeigen eine für das Dynesys-System der Anmelderin ausgebildete, zum Stand der Technik gehörende Pedikelschraube. Der einen kreisförmigen Querschnitt aufweisende Schaft 12 ist mit einem Gewinde 31 versehen und konisch ausgebildet. Der Kopf 11 ist auf zwei einander gegenüberliegenden Seiten mit Haltemulden 51 versehen, die während der Implantation zum Halten der Schraube mittels eines entsprechend ausgebildeten Instrumentes dienen. Auf den anderen beiden einander gegenüberliegenden Seiten ist der Kopf 11 abgeflacht. Die hierdurch gebildeten ebenen Stützflächen 45 dienen zur Abstützung von nicht dargestellten, zylindrischen komprimierbaren Druckkörpern des Stabilisierungssystems. Ein auf Zug vorspannbares Band des Stabilisierungssystems wird durch diesen elastischen Druckkörper und durch einen im Kopf 11 ausgebildeten Durchgang 47 hindurch geführt und mittels einer nicht dargestellten Fixierschraube, die in ein im Kopf 11 ausgebildetes Gewinde 49 eingeschraubt wird, am Kopf 11 fixiert. Im implantierten Zustand ist das Band auf Zug vorgespannt, während der zwischen zwei Schraubenköpfen 11 angeordnete und sich an den Stützflächen 45 abstützende elastische Druckkörper zusammengedrückt ist. Hierdurch wird insgesamt ein dynamisches, sowohl auf Druck- als auch auf Zugbelastungen senkrecht zur Schraubenachse elastisch reagierendes Stabilisierungssystem realisiert.

Fig. 2 zeigt schematisch eine solche bekannte Pedikelschraube, die in einen Wirbel implantiert ist und sich hierbei mit ihrem Schaft 12 durch den Pedikel 13 hindurch bis in den Wirbelkörper 15 erstreckt. Wie eingangs erläutert, neigen starre Pedikelschrauben unter der Einwirkung einer über den Kopf 11 aufgebrachten intervertebralen Kraft F zu einem Verkippen oder Drehen um einen so genannten Togglepoint oder Drehpunkt 53, der - wie Untersuchungen ergeben haben - im Übergangsbereich 23 zwischen Pedikel 13 und Wirbelkörper 15 liegt.

Unter Berücksichtigung der charakteristischen Bereiche des Wirbels sowie der durch umfangreiche Tests ermittelten Lage des Togglepoints 53 gelten sowohl für die bekannten Pedikelschrauben gemäß Fig. 2 als auch für die nachstehend beschriebenen Befestigungselemente die in der nachstehenden Tabelle angegebenen Minimal-, Maximal- und Durchschnittsabmessungen (Angaben jeweils in mm). Folgende Parameter sind in der Tabelle aufgeführt, wobei mit "Band" das vorstehend erwähnte, auf Zug vorspannbare, durch die in den Köpfen 11 ausgebildeten Durchgänge 47 und die zwischen den Köpfen 11 befindlichen Druckkörper hindurch geführte Band des Stabilisierungssystems gemeint ist:
- Lges:: axiale Länge von Bandachse 55 bis Schraubenspitze 57
- L1:: axiale Länge von Bandachse 55 bis Togglepoint 53
- L2:: axiale Länge von Togglepoint 53 bis Schraubenspitze 57
- L3:: axiale Länge von Bandachse 55 bis Knocheneintritt 59
- L4:: axiale Länge von Knocheneintritt 59 bis Togglepoint 53
- D1:: Durchmesser an Schraubenspitze 57
- D2:: Durchmesser an Togglepoint 53
- D3:: Durchmesser an Knocheneintritt 59

| [mm] | Minimum | Maximum | Durchschnitt |
|---|---|---|---|
| Lges | 40 | 60 | 50 |
| L1 | 8 | 40 | 30 |
| L2 | 0 | 40 | 20 |
| L3 | 6 | 15 | 10 |
| L4 | 10 | 30 | 20 |
| D1 | 2,8 | 5,5 | 3,4 |
| D2 | 4 | 6,75 | 4,9 |
| D3 | 5,2 | 8 | 6,4 |

Die in einem konkreten Fall zu verwendenden Maße sind unter anderem von der bei der Positionierung der Pedikelschrauben verwendeten Technik abhängig.

Die in Richtung der Längsachse des implantierten Befestigungselementes gemessene Biegesteifigkeit C kann definiert werden als das Produkt aus Elastizitätsmodul und Flächenmoment zweiter Ordnung (Flächenträgheitsmoment). Das Biegesteifigkeitsprofil des Befestigungselementes eignet sich besonders gut dazu, den Unterschied zwischen herkömmlichen starren Pedikelschrauben einerseits und den hier angegebenen flexiblen Befestigungselementen andererseits aufzuzeigen. Die Biegesteifigkeit des Befestigungselementes lässt sich durch die Wahl des Materials für den Schaft und durch die Schaftgeometrie beeinflussen. Im ersten Fall wird der Elastizitätsmodul variiert, wohingegen im zweiten Fall das Flächenmoment verändert wird.

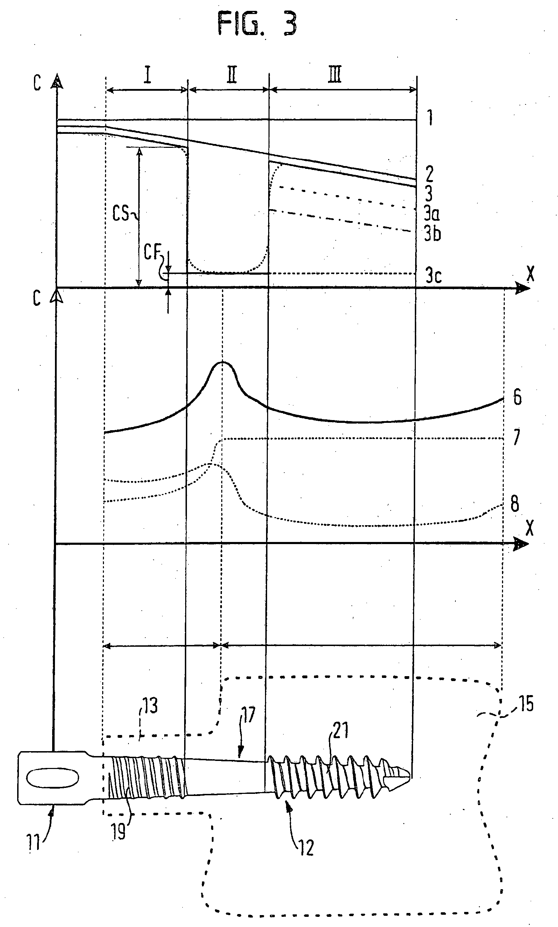

Fig. 3 zeigt im unteren Drittel ein Befestigungselement, das in Form einer Pedikelschraube vorgesehen ist und einen Kopf 11 sowie einen Schaft 12 aufweist. Der Schaft 12 ist mit einer Entlastungszone 17 (Zone II) versehen. Auf Einzelheiten zu dieser Entlastungszone 17 wird an dieser Stelle nicht näher eingegangen. Konkrete Ausführungsformen sind nachstehend erläutert. Fig. 3 dient noch zur Erläuterung allgemeiner Zusammenhänge.

Im oberen Drittel der Fig. 3 sind Biegesteifigkeitsprofile für unterschiedlich ausgebildete Befestigungselemente dargestellt. Das mittlere Drittel der Fig. 3 zeigt das Biegesteifigkeitsprofil der Knochenstruktur, in welche die Pedikelschraube implantiert ist, d.h. die Knochenstruktur aus Pedikel 13 und Wirbelkörper 15. Die dargestellten Biegesteifigkeitsprofile sind entlang der Mittelachse des Schaftes 12 des Befestigungselementes gemessen, die mit der Mittelachse des Pedikels 13 zumindest näherungsweise zusammenfällt.

Die Darstellung der Fig. 3 ist in drei Zonen längs des Schaftes 12 unterteilt. Im Folgenden wird der Schaftbereich zwischen dem Kopf 11 und dem Beginn der Entlastungszone 17 auch als oberer Schaftbereich 19 (Zone I) bezeichnet, während der vom Kopf 11 aus gesehen hinter der Entlastungszone 17 gelegene Bereich des Schaftes 12 auch als unterer Schaftbereich 21 (Zone III) bezeichnet wird.

Kurve 1 zeigt das Biegesteifigkeitsprofil einer herkömmlichen, als ideal zylindrisch angenommenen starren Pedikelschraube ohne Entlastungszone. Die Biegesteifigkeit C ist über die gesamte Schaftlänge konstant.

Kurve 2 zeigt das Biegesteifigkeitsprofil einer sich in Richtung des freien Schaftendes verjüngenden konischen Pedikelschraube bzw. einer Pedikelschraube mit konischem Kern. Die Biegesteifigkeit nimmt in diesem Fall stetig ab.

Die Kurven 3, 3a, 3b und 3c zeigen Biegesteifigkeitsprofile von Befestigungselementen, die jeweils mit einer Entlastungszone bzw. einem flexiblen Bereich 17 versehen sind. Die Entlastungszone 17 beginnt vor dem Übergang zwischen Pedikel 13 und Wirbelkörper 15 und endet hinter diesem Übergang innerhalb des Wirbelkörpers 15. Das Biegesteifigkeitsprofil weist jeweils einen topfartigen Verlauf mit steilen Wänden auf, d.h. die Abnahme bzw. Zunahme der Biegesteifigkeit erfolgt abrupt. Dabei können die Übergänge mehr oder weniger stark stufenförmig ausgeprägt sein. Durch die gepunktete Kurve ist ein Beispiel gezeigt, bei dem die Übergänge gegenüber dem mit durchgezogener Linie gezeigten Verlauf abgerundet sind.

Die Kurven 3a und 3b zeigen Biegesteifigkeitsprofile, die jeweils dem Verlauf der Kurve 3 mit dem Unterschied entsprechend, dass der untere Schaftbereich 21 (Zone III) mit einer signifikant reduzierten Steifigkeit versehen ist, die aber immer noch deutlich über derjenigen in der Entlastungszone 17 (Zone II) liegt.

In dem Beispiel der Kurve 3c entspricht die Biegesteifigkeit im unteren Schaftbereich 21 demjenigen in der Entlastungszone 17. Mit anderen Worten: in diesem Beispiel erstreckt sich die Entlastungszone 17 bis zur Spitze am freien Ende des Befestigungselementes.

Das obere Drittel der Fig. 3 zeigt ferner, dass die Biegesteifigkeit CF in der Entlastungszone 17 (Zone II) nur etwa ein Zehntel des entsprechenden Wertes CS im oberen Schaftbereich 19 (Zone I) beträgt.

Im mittleren Drittel der Fig. 3 zeigt Kurve 7 den axialen Verlauf des Elastizitätsmoduls der Knochenstruktur aus Pedikel 13 und Wirbelkörper 15. Der entsprechende Verlauf des Flächenmoments zweiter Ordnung des Knochens ist in Kurve 8 gezeigt. Mit einer durchgezogenen Linie ist als Kurve 6 die Summe aus den beiden Kurven 7 und 8 dargestellt, die in diesem vereinfachten Modell das Profil der Biegesteifigkeit C der Knochenstruktur aus Pedikel 13 und Wirbelkörper 15 darstellt.

Hieraus ergibt sich, dass die Biegesteifigkeit des Wirbels im Bereich des Übergangs zwischen Pedikel 13 und Wirbelkörper 15 am größten ist. Der Vergleich mit dem oberen Drittel der Fig. 3 zeigt, dass die Entlastungszone 17 derart im Schaft 12 positioniert sein kann, dass bei implantiertem Befestigungselement entsprechend dem unteren Drittel der Fig. 3 der Übergang zwischen Pedikel 13 und Wirbelkörper 15 und damit der Bereich maximaler Biegesteifigkeit des Wirbels innerhalb der Entlastungszone 17 des Schaftes 12 und insbesondere - in axialer Richtung gesehen - mittig in der Entlastungszone 17 gelegen ist.

Mögliche Längen für die in Fig. 3 dargestellten Zonen I, II und III des Schaftes 12 des Befestigungselementes lauten wie folgt:
Die axiale Länge der Zone I und somit etwa der Abstand von der Unterseite des Kopfes 11 bis zum Beginn der Entlastungszone 17 kann zwischen 8 mm und 35 mm liegen. Die kleinste Länge kann für den Wirbel L5 zur Anwendung kommen, wenn die Schraube so weit eingeschraubt wird,
dass der Schraubenkopf 11 an dem Knochen anliegt. Die größte Länge kommt zur Anwendung, wenn mit einer so genannten posterior-medialen Schraubensetztechnik gearbeitet wird, wobei das System posterior der Facettengelenke positioniert wird.

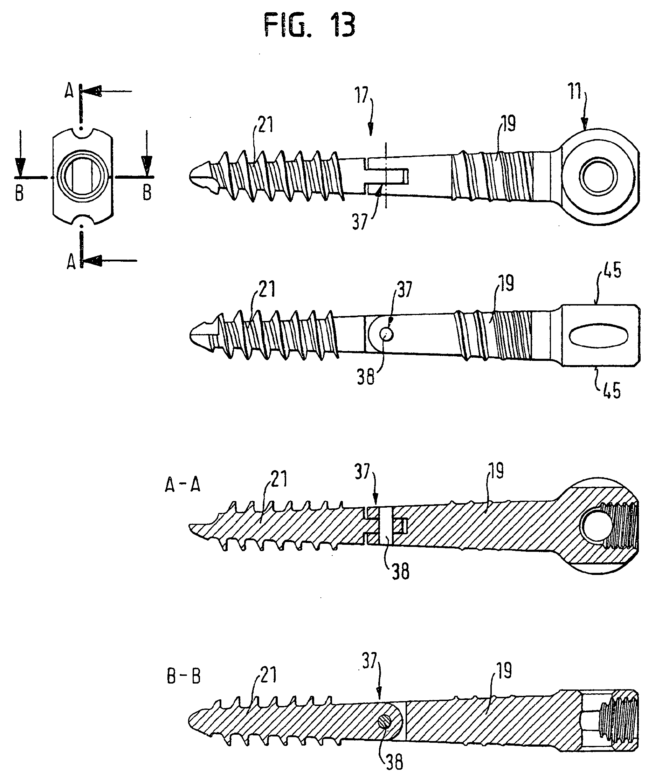

Für die Länge der Zone II, d.h. der Entlastungszone oder flexiblen Übergangszone 17, kann ein Bereich von 0 mm bis 35 mm vorgesehen sein. Die kleinste Länge kann dann vorliegen, wenn die Entlastungszone 17 als Gelenk ausgeführt ist (vgl. nachstehendes Ausführungsbeispiel in Fig. 13). In diesem Fall wird die Entlastungszone 17 praktisch von der Drehachse des Gelenks gebildet. Die größte Länge kann dann vorliegen, wenn die Entlastungszone 17 sich bis zur Spitze des Befestigungselementes erstreckt.

Für die Länge des unteren Schaftbereichs 21 kann ein Bereich von 0 mm bis 35 mm vorgesehen sein. Die kleinste Länge ist dann gegeben, wenn kein unterer Schaftbereich 21 im Sinne der Fig. 3 vorgesehen ist, sondern sich die Entlastungszone 17 bis zur Spitze des Entlastungselements erstreckt. Die größte Länge kann dann vorliegen, wenn das freie Ende des Befestigungselementes den anterioren Kortex berührt oder sogar penetriert (so genannte bikortikale Schraubensetztechnik).

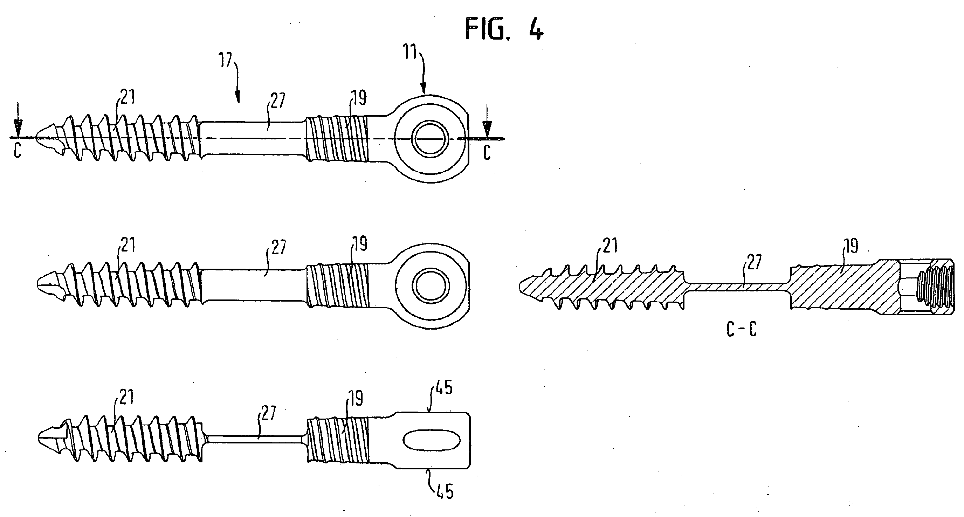

Bei dem Ausführungsbeispiel einer Pedikelschraube in Fig. 4 ist die Entlastungszone 17 durch Materialwegnahme am Schaft gebildet. Folglich ist hier die Entlastungszone 17 ein Schaftbereich 27 mit gegenüber den angrenzenden Schaftbereichen 19, 21 reduzierter Querschnittsfläche. Dabei ist der Querschnitt in der Entlastungszone 17 nicht kreisförmig, sondern der Schaftbereich 27 ist blattfederartig ausgebildet und in Form eines dünnen Streifens vorgesehen. Die Breite des Streifens entspricht dem ursprünglichen Schaftdurchmesser, wohingegen die Streifendicke wesentlich kleiner als der ursprüngliche Schaftdurchmesser ist.

In diesem Beispiel ist das Biegesteifigkeitsprofil des Schaftes somit bezogen auf dessen Längsachse rotationsunsymmetrisch. Dabei ist die Orientierung der Entlastungszone 17 derart gewählt, dass die von dem Streifen 27 definierte Ebene parallel zu den ebenen Stützflächen 45 am Schraubenkopf 11 verläuft. Die Entlastungszone 17 ist daher nur in einer Ebene voll wirksam, wobei diese Ebene von der Längsachse des Schaftes und einer Normalen der ebenen Stützflächen 45 aufgespannt ist.

Eine erste Grobdimensionierung einer Schraube gemäß Fig. 4 hat folgendes ergeben: Den Ausgangspunkt bildete eine herkömmliche Pedikelschraube (ohne Entlastungszone) des Dynesys-Systems der Anmelderin, deren Material (eine Titanlegierung) einen Elastizitätsmodul von E = 105.000 N/mm² aufweist. Unter Beachtung des Dauerfestigkeitskriteriums (dynamische Belastbarkeit bzw. Biegewechselsteifigkeit unter maximaler Deformation von 10°) ergaben sich folgende Durchschnittsmaße für die Entlastungszone 17:

| | |
|---|---|
| Länge: | 15 mm |
| Breite: | 6,5 mm |
| Höhe: | 1 mm |

Überschlagsrechnungen ergaben, dass mit einer derartigen Schraube lediglich etwa 10% der maximal zulässigen Flächenpressung im Wirbelkörper hervorgerufen werden kann. Mit anderen Worten vereinfacht ausgedrückt: eine solche Schraube ist weniger stabil, als es der Wirbelkörper erlauben würde.

Dieses Berechnungsbeispiel - das also nicht zu einem optimalen Befestigungselement führt, sich gleichwohl aber für die Erläuterung des Grundprinzips der hier angegebenen Pedikelschraube eignet - zeigt also, dass die Entlastungszone einerseits die Belastbarkeit des Befestigungselementes und andererseits die Belastung des Wirbelkörpers bestimmt: Einerseits sollte die Biegesteifigkeit in der Entlastungszone nur so weit reduziert werden, dass die Belastbarkeit des Wirbelkörpers gerade nicht überschritten wird, denn je biegesteifer die Entlastungszone ausgeführt ist, desto größere Kräfte bzw. Momente können in den Wirbelkörper übertragen werden, was grundsätzlich gewünscht ist, denn der freie Endbereich des Befestigungselementes soll maximal mittragen, nur eben ohne den Wirbelkörper zu überlasten (und so eine Lockerung hervorzurufen). Unter Beachtung der maximalen Belastbarkeit des Wirbelkörpers wird einerseits also eine möglichst hohe Biegesteifigkeit in der Entlastungszone angestrebt. Andererseits wird mit zunehmender Biegesteifigkeit der Entlastungszone die Belastbarkeit des Befestigungselementes früher erreicht.

Das Befestigungselement wird folglich derart ausgelegt, dass sich ein optimaler "Kompromiss" zwischen diesen beiden im Grunde gegenläufigen Optimierungskriterien einstellt.

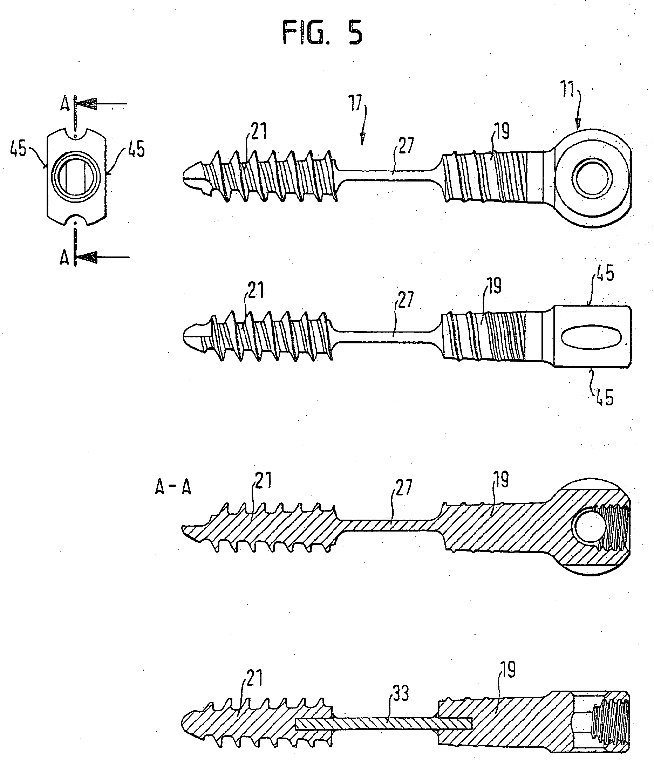

In dem Beispiel der Fig. 5 ist abweichend von der in Fig. 4 dargestellten Ausgestaltung der die Entlastungszone 17 bildende Schaftbereich 27 mit einem kreisförmigen Querschnitt versehen. Das Biegesteifigkeitsprofil der Schraube ist hier somit bezogen auf deren Längsachse rotationssymmetrisch.

In der unteren Darstellung in Fig. 5 ist eine Variante gezeigt, bei welcher der die Entlastungszone 17 bildende Schaftbereich nicht einstückig ausgeführt, sondern von einem separaten Zwischenstück 33 gebildet ist, das in den oberen und unteren Schaftbereichen 19, 21 verankert ist. Bei dem Zwischenstück 33 kann es sich um einen Stab aus einem Material von besonders geringer Biegesteifigkeit handeln ("superelastisches" Material). Insbesondere handelt es sich um ein Material, das sich bis zu 10% oder mehr ohne bleibende Verformung deformieren lässt, was von den üblichen Implantatstählen oder von Titan nicht erreicht wird. Für das Zwischenstück 33 in Frage kommt insbesondere ein Memory-Metall auf NiTi-Basis (z.B. Nitinol).

Es ist auch möglich, als Zwischenstück 33 ein drahtseilartig ausgebildetes Element vorzusehen.

Während bei einer Schwächung des Schaftes durch Materialwegnahme die Querschnittsfläche reduziert und damit das Flächenmoment des Schaftes verringert wird, kann durch eine Änderung des Materials in der Entlastungszone 17 der Elastizitätsmodul des Schaftes gezielt verändert werden.

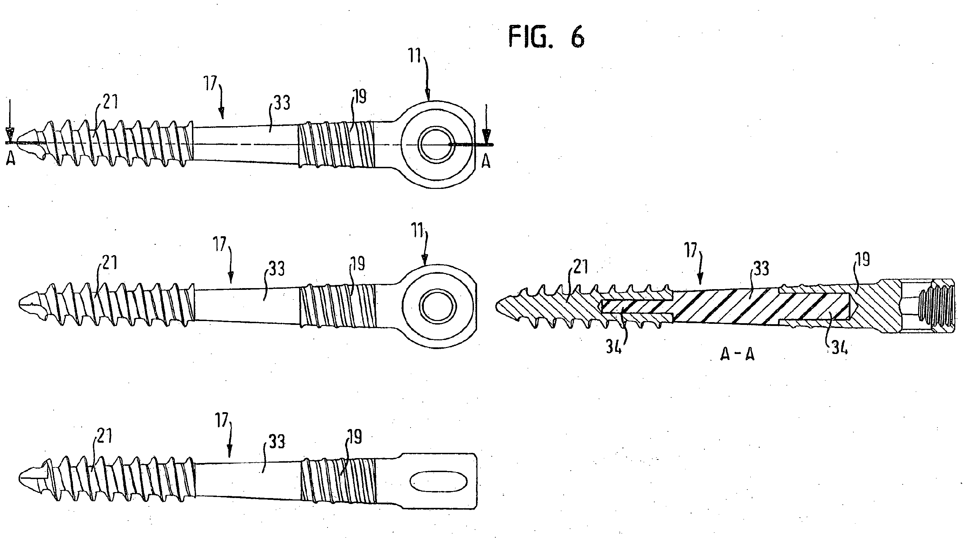

Ein weiteres Beispiel für eine solche Reduzierung des Elastizitätsmoduls durch Materialänderung zeigt Fig. 6. Anders als bei dem Beispiel in der unteren Darstellung in Fig. 5 erfolgt hier keine Querschnittsreduzierung, sondern ausschließlich eine Materialänderung.

Als Material für die hier angegebene Pedikelschraube und damit für die unteren und oberen Schaftbereiche 19, 21 und den Schraubenkopf 11 wird insbesondere eine biokompatible Titan- oder Stahllegierung verwendet. Für ein hier die Entlastungszone 17 bildendes separates Zwischenstück 33 wird ein Material verwendet, das einen deutlich niedrigeren Elastizitätsmodul als das Schaftmaterial aufweist und damit für eine entsprechende Flexibilität bzw. Biegsamkeit des Schaftes sorgt. Ein mögliches Material für das Zwischenstück 33 ist Kunststoff, insbesondere ein Elastomer. Der Kunststoff kann faserverstärkt sein. Durch die Materialwahl und damit die Vorgabe des Elastizitätsmoduls kann die Biegesteifigkeit des Schaftes prinzipiell beliebig eingestellt werden.

Wie die Querschnittsdarstellung rechts in Fig. 6 zeigt, ist das entsprechend dem Schaftverlauf konisch ausgebildete Zwischenstück 33 mit sich zu beiden Seiten in axialer Richtung erstreckenden Verankerungsfortsätzen 34 versehen, über welche das Zwischenstück 33 an den angrenzenden Schaftbereichen 19, 21 befestigt ist.

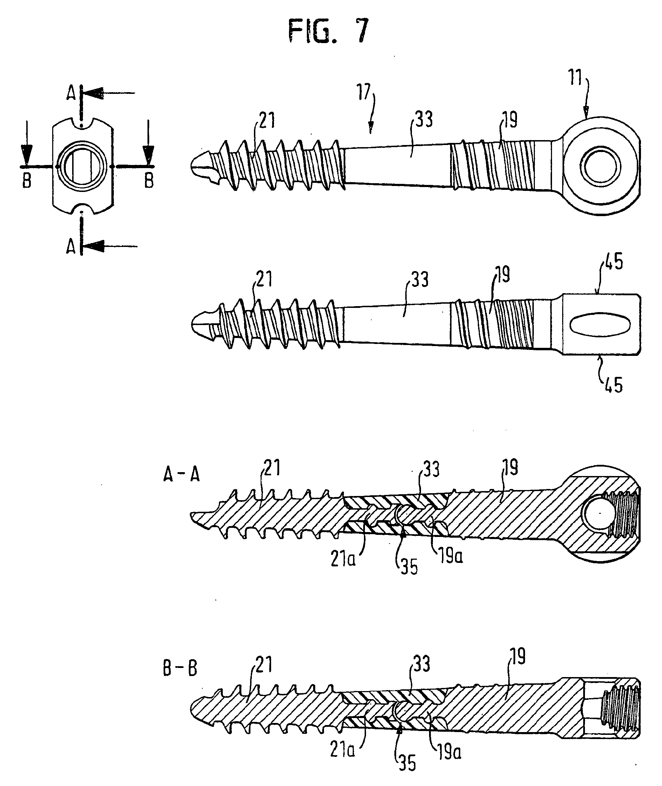

Fig. 7 zeigt eine Variante, bei der ebenfalls ein beispielsweise aus Kunststoffmaterial hergestelltes Zwischenstück 33 zur Bildung der Entlastungszone 17 vorgesehen ist. Die Verankerung an den angrenzenden Schaftbereichen 19, 21 erfolgt durch einstückig an diesen Schaftabschnitten 19, 21 ausgebildete axiale Fortsätze 19a, 21a, die zur Verbesserung des Formschlusses mit radialen Verbreiterungen versehen sind.

Eine Besonderheit dieser Variante besteht darin, dass die Fortsätze 19a, 21a etwa in der Mitte der Entlastungszone 17 innerhalb des Zwischenstücks 33 gelenkartig zusammenwirken, in dem hier dargestellten Beispiel nach dem Kugel/Schale-Prinzip, wobei alternativ beispielsweise eine Scharnieranordnung vorgesehen sein kann. Der obere Schaftabschnitt 19 und der untere Schaftabschnitt 21 sind in diesem Beispiel also durch ein Gelenk 35 miteinander verbunden, das von dem elastischen Material des Zwischenstücks 33 umgeben ist, wodurch im Fall einer Auslenkung des Schaftes für die entsprechenden Rückstellkräfte gesorgt ist. Der die Entlastungszone 17 bildende Gelenkbereich kann insbesondere mit dem das Zwischenstück 33 bildenden Material umspritzt sein.

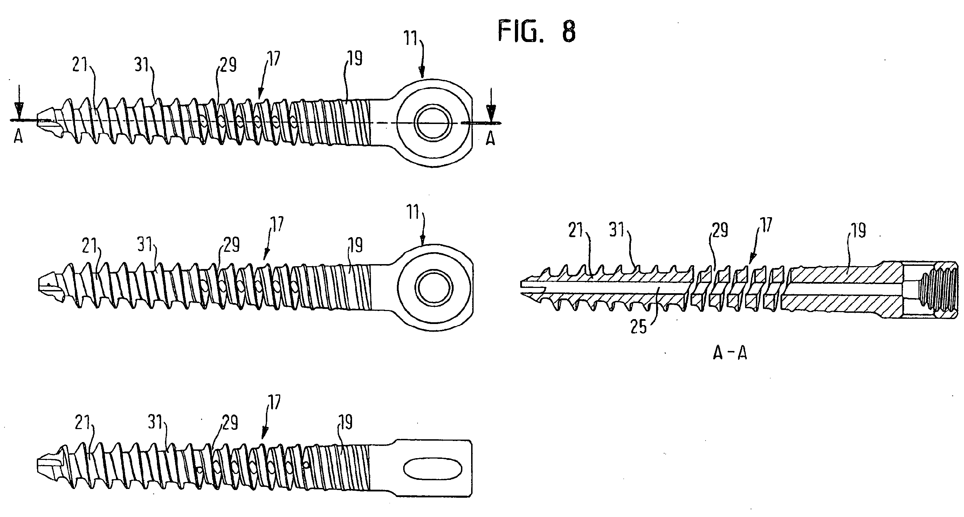

Die Fig. 8, 9 und 10 zeigen Ausführungsformen, bei denen wiederum das Flächenmoment reduziert und gleichzeitig das Schaftmaterial optimal ausgenutzt ist.

In dem Beispiel der Fig. 8 ist der Schaft mit einer zentralen Bohrung 25 versehen. Dies ist fertigungstechnisch von Vorteil und erlaubt es außerdem, die Pedikelschrauben mit Hilfe eines Kirschnerdrahtes zu implantieren, was insbesondere bei perkutaner Implantation vorteilhaft ist.

Anders als bei den vorstehend erläuterten Beispielen ist hier das Gewinde 31 der Schraube durchgehend ausgeführt. Im Bereich der Entlastungszone 17 ist die Wandung des Schaftes im Gewindetal durchbrochen. Hierdurch ist der Schaft im Bereich der Entlastungszone 17 nach Art einer Schraubenfeder oder eines Korkenziehers ausgebildet. Eine solche Entlastungszone lässt sich beispielsweise durch Drahterodieren oder durch einen angetriebenen Scheibenfräser problemlos fertigen.

Der in der Entlastungszone im Gewindetal umlaufende Schlitz kann eine deutlich geringere Schlitzbreite aufweisen als in Fig. 8 gezeigt. Insbesondere kann die Schlitzbreite derart klein gewählt werden, dass bei einer gewissen Durchbiegung des Schaftes die Windungen in der Entlastungszone aneinander stoßen bzw. aufliegen, so dass hierdurch Druckkräfte übertragen werden können.

Ferner ist es möglich, dass sich der gemäß Fig. 8 nur in der Entlastungszone 17 ausgebildete Schlitz bis hin zur Schraubenspitze erstreckt, d.h. im Anschluss an den oberen Schaftbereich 19 der gesamte verbleibende Schaft nach Art einer Schraubenfeder oder eines Korkenziehers ausgebildet ist.

In einer weiteren Ausgestaltung können die Schlitze mit einem bioresorbierbaren Stoff gefüllt werden, der die Federwirkung reduziert oder aufhebt. Hierdurch kann die intraoperative Stabilität des Schaftes erhöht werden. Je nach Ausgestaltung des Füllstoffes erfolgt die Resorption vergleichsweise schnell oder innerhalb einiger Tage. Im Anschluss an die Resorption ist dann die durch die Schaftgeometrie vorgegebene Flexibilität der Schraube voll wirksam.

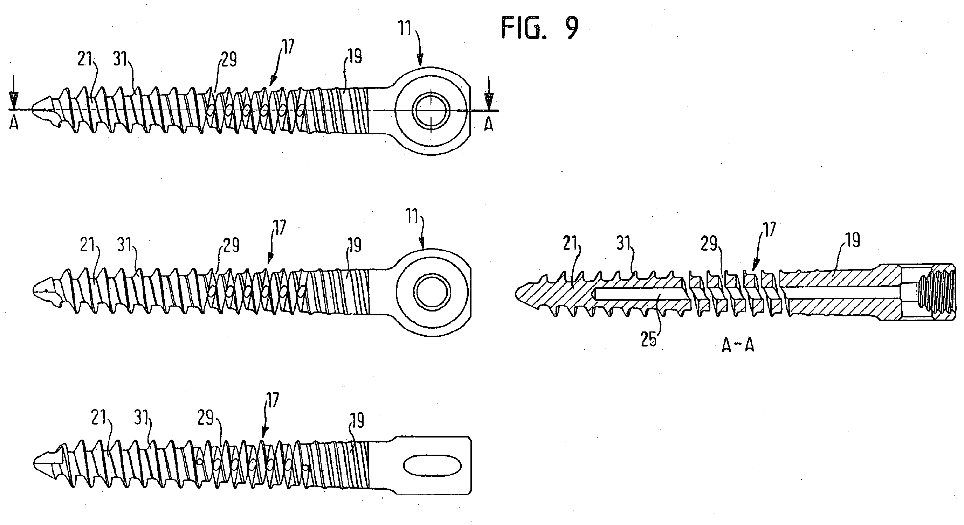

Während in dem Beispiel der Fig. 8 der in der Schaftwandung ausgebildete Schlitz gleichsinnig mit dem Gewinde 31 ausgeführt ist, kann gemäß dem Beispiel der Fig. 9 der Schlitz auch gegenläufig zum Gewinde 31 ausgebildet werden. Beim Eindrehen der Schraube kommt es durch die dabei wirksamen Reibkräfte zu einer Einschnürung des Schaft- oder Kerndurchmessers im Bereich der Entlastungszone 17. Das Zurückfedern des Schaftes nach Wegfall der Eindrehkraft bewirkt eine weitere Kompression des umliegenden Knochenmaterials, was die Primärstabilität der Schraube deutlich verbessern kann.

Das Vorsehen einer durchgehenden Zentralbohrung, wie im Beispiel der Fig. 8, ist nicht zwingend erforderlich. Wie das Beispiel der Fig. 9 zeigt, kann auch eine sich nur teilweise durch den Schaft hindurch erstreckende zentrale Bohrung 25 vorgesehen sein. In diesem Beispiel endet die Bohrung 25 im Bereich des unteren Schaftabschnitts 21.

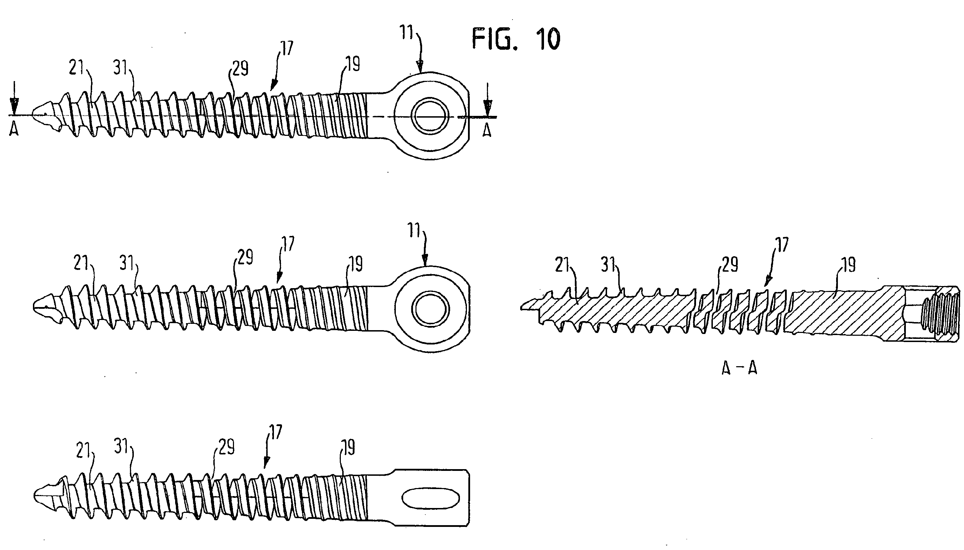

Wie das Beispiel der Fig. 10 zeigt, ist es auch bei Schäften ohne zentrale Bohrung möglich, spiral- oder wendeiförmig umlaufende Vertiefungen vorzusehen, um die Entlastungszone 17 zu bilden. Durch eine entsprechend gewählte Einstichtiefe können auch in diesem Fall eine Wendel ausbildende Strukturen hergestellt werden, die insofern eine zentrale Bohrung überflüssig machen. Die Orientierung der Vertiefungen bzw. Schlitze kann hierbei entsprechend den Beispielen der Fig. 8 und 9 entweder gleich- oder gegenläufig in Bezug auf das Schraubengewinde 31 gewählt werden.

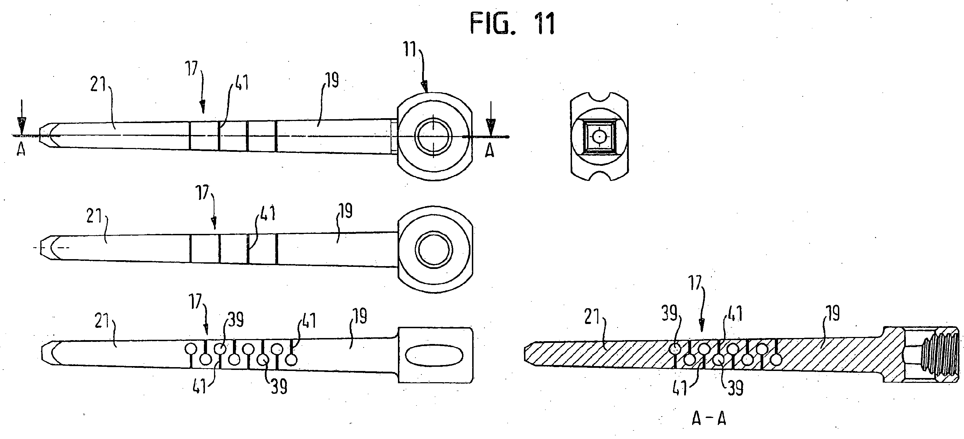

Das Ausführungsbeispiel der Fig. 11 umfasst mehrere Besonderheiten, die nachstehend erläutert werden, wobei eine solche Kombination von Merkmalen nicht zwingend ist, sondern die einzelnen Aspekte auch unabhängig voneinander in Verbindung mit anderen Ausgestaltungen realisiert werden können.

Bei dem Befestigungselement der Fig. 11 handelt es sich nicht um eine Schraube, sondern um ein stiftartiges Befestigungselement, das nicht eingedreht, sondern in den Wirbel eingeschlagen wird.

Zur Bildung der Entlastungszone 17 ist der Schaft mit versetzt angeordneten Schlitzen 41 geringer Breite versehen, die jeweils in eine durchgehende Bohrung 39 münden. Aufgrund der geringen Schlitzbreite können die zum Einschlagen erforderlichen Schlagimpulse problemlos in axialer Richtung übertragen werden.

Der Querschnitt des Schaftes kann von einer Kreisform abweichen und insbesondere oval oder elliptisch ausgeführt sein, um hierdurch eine bessere Anpassung an die natürliche Pedikelform zu erzielen, wie es eingangs bereits erläutert wurde.

Des Weiteren kann der Schaft des einzuschlagenden Befestigungsstiftes mit einem im Ansatz vorhandenen Gewinde versehen sein, um für den Fall einer Re-Operation das Explantieren des Befestigungselementes zu erleichtern.

Es sei ausdrücklich bemerkt, dass auch mit einer schlitz- oder nutförmigen, wendel- oder spiralförmig umlaufenden Vertiefung versehene Schrauben, wie sie vorstehend erläutert wurden, einen Schaft mit einem von einer Kreisform abweichenden Querschnitt aufweisen können, d.h. nicht rotationssymmetrische Schaftgeometrien sind nicht auf Einschlagstifte gemäß Fig. 11 beschränkt.

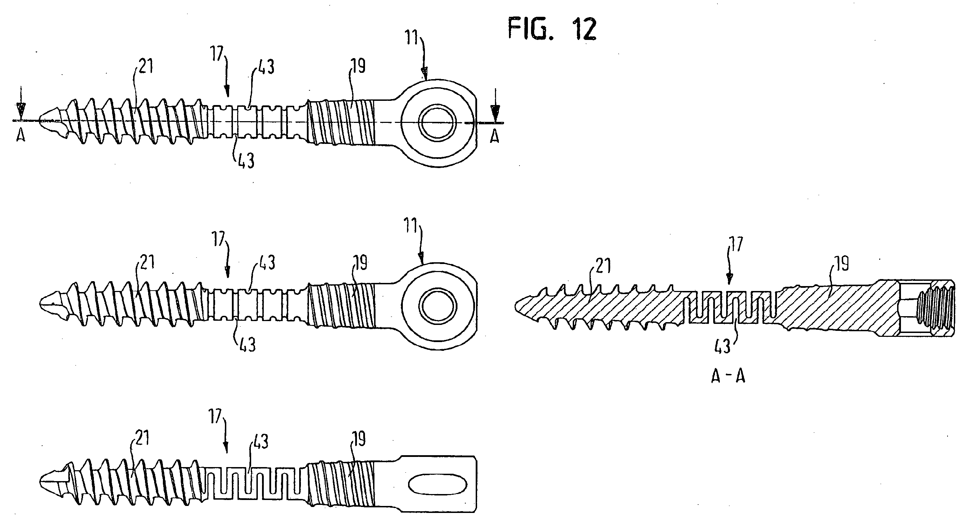

Fig. 12 zeigt ein Beispiel mit versetzt angeordneten Entlastungsschlitzen oder -kerben 43, deren Breite jeweils größer ist als diejenige der Schlitze 41 im Beispiel der Fig. 11. Derartige Entlastungskerben können sowohl bei Schäften mit kreisförmigem als auch mit einem von einer Kreisform abweichenden Querschnitt vorgesehen sein.

Es ist des Weiteren möglich, die Biegesteifigkeit des Schaftes auf Null zu reduzieren. Hierzu kann beispielsweise entsprechend der Ausführungsform der Fig. 13 ein Drehgelenk 37 zwischen dem oberen Schaftabschnitt 19 und dem unteren Schaftabschnitt 21 vorgesehen sein. Ein die Drehachse definierender Gelenkstift 38 des Gelenks 37 verläuft dabei parallel zu den ebenen Stützflächen 45 des Schraubenkopfes 11. Die Lage des Gelenks 37 entlang der Schaftachse kann so gelegt werden, dass die Drehachse des Gelenks 37 bei implantierter Schraube im Bereich des vorstehend erwähnten Togglepoints (Fig. 3) liegt und insbesondere durch den Togglepoint läuft.

Im Fall eines solchen Drehgelenks 37 ist keine sich über eine signifikante axiale Länge des Schaftes erstreckende Entlastungszone im Sinne der vorstehend beschriebenen Ausführungsbeispiele vorhanden. Im Beispiel der Fig. 13 ist es vielmehr das Gelenk 37 selbst, das die Entlastungszone 17 bildet.

Des Weiteren ist es möglich, die Entlastungszone derart auszubilden, dass deren Biegesteifigkeit im Laufe der Zeit zunimmt. Beispielsweise kann für die Entlastungszone ein Kunststoffmaterial verwendet werden, dessen Härte sich im Laufe der Zeit erhöht, wodurch die Kraft- oder Momentübertragung auf den freien Endbereich des Befestigungselementes entsprechend zunimmt. Hierdurch kann dem Umstand Rechnung getragen werden, dass nach einer bestimmten Zeit im Anschluss an die Operation davon ausgegangen werden kann, dass das Befestigungselement gut in das Knochenmaterial eingewachsen ist. Die anfangs noch relativ hohe Flexibilität des Schaftes dient hierbei also insbesondere dem Zweck, hohe Belastungen kurz nach der Operation zu vermeiden.

Alternativ kann - wie vorstehend bereits in Verbindung mit dem Beispiel der Fig. 8 erwähnt - der Schaft derart ausgebildet werden, dass die Biegesteifigkeit in der Entlastungszone im Laufe der Zeit abnimmt. Hierzu können beispielsweise resorbierbare Bestandteile vorgesehen werden, die bei der Implantation eine versteifende Wirkung besitzen und im Laufe der Zeit resorbiert werden, wodurch sich die Flexibilität des Schaftes im Bereich der Entlastungszone nach und nach erhöht.

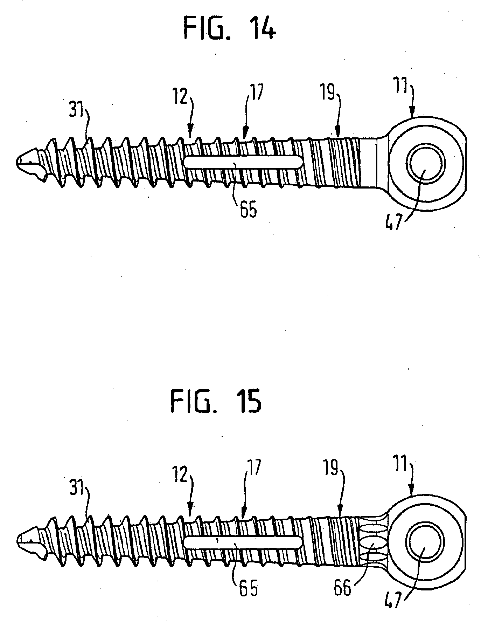

Fig. 14 zeigt eine Pedikelschraube, deren Schaft 12 zur Bildung der Entlastungszone 17 einen den Schaft 12 durchdringenden und in Längsrichtung des Schaftes 12 verlaufenden Schlitz 65 aufweist.

In Fig. 15 ist am Beispiel der Pedikelschraube von Fig. 14 gezeigt, dass der obere Schaftbereich 19 in Längsrichtung verlaufende Vertiefungen 66 an der Oberfläche aufweisen kann, in die der Knochen einwachsen kann, wodurch eine Verdrehsicherung geschaffen wird.

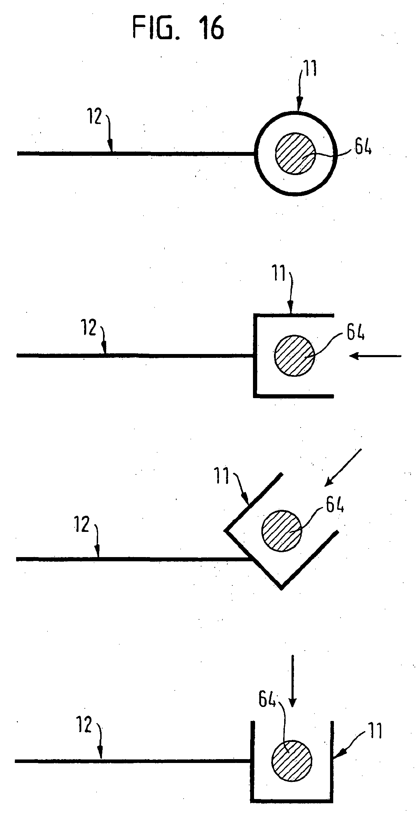

In Fig. 16 sind - rein schematisch - mehrere mögliche Ausgestaltungen des Kopfes 11 eines Befestigungselementes der hier angegebenen Art dargestellt, die sich dadurch unterscheiden, wie ein Verbindungselement 64 eines intervertebralen Stabilisierungssystems - beispielsweise ein Band 64 in dem Dynesys-System der Anmelderin (siehe auch nachstehende Beschreibung der Fig. 17) - im bzw. am Kopf 11 aufgenommen werden kann.

Während bei der oberen Ausführungsform der Kopf 11 als Ring oder Öse ausgebildet ist, durch den bzw. die das Verbindungselement 64 hindurchgeführt wird, ist bei den anderen Ausführungsformen der Kopf 11 nach Art eines - bezogen auf die Längsachse des Befestigungselementes - aufrecht stehenden, geneigten oder auf der Seite liegenden "U" ausgeführt, so dass das Verbindungselement 64 nicht durch eine Öffnung "hindurch gefädelt" zu werden braucht, sondern (in Pfeilrichtung) eingelegt werden kann, und zwar entweder von oben (so genanntes "Toploading"-Prinzip) oder von der Seite (so genanntes "Sideloading"-Prinzip). Alle vorstehend insbesondere anhand der Fig. 3 bis 15 beschriebenen Ausführungsbeispiele der hier angegebenen Pedikelschraube können grundsätzlich mit jeder der in Fig. 16 gezeigten Kopf-Varianten versehen werden.

Das "Toploading"-Prinzip ist beispielsweise in EP 528 706 beschrieben.

Es ist im Stand der Technik verbreitet, das Verbindungselement 64 durch eine insbesondere in Einlegerichtung in ein am Kopf 11 angeordnetes Gewinde eingeschraubte Schraube zu fixieren.

Fig. 17 zeigt in mehreren Darstellungen ein Beispiel für ein intervertebrales Stabilisierungssystem, das hier angegebene Befestigungselemente, insbesondere in Form von Pedikelschrauben, beinhalten kann. Bei dem gezeigten dynamischen System handelt es sich um ein elastisches Stützsystem (Dynesys-System der Anmelderin), wie es vorstehend bereits mehrfach erwähnt wurde.

Benachbarte Wirbel sind durch zwei baugleiche Teilsysteme miteinander verbunden. In jeden Wirbel sind zwei jeweils einen Schaft 12 und einen Kopf 11 aufweisende Pedikelschrauben implantiert, die sich jeweils durch ein Pedikel 13 hindurch in den Wirbelkörper 15 erstrecken. In beiden Teilsystemen ist jeweils zwischen zwei Schraubenköpfen 11 ein komprimierbarer Druck- oder Stützkörper 63 angeordnet. Ein im implantierten Zustand auf Zug vorgespanntes und mittels Fixierschrauben 61 in den Köpfen 11 fixiertes Band 64 erstreckt sich durch den Druckkörper 63 und die Köpfe 11 hindurch. Mit dem Band 64 werden Zugkräfte und mit dem Druckkörper Druckkräfte elastisch aufgenommen. Bei den gezeigten Pedikelschrauben kann es sich um Befestigungselemente der hier angegebenen Art handeln.

### Bezugszeichenliste

- 11: Kopf
- 12: Schaft
- 13: Pedikel
- 15: Wirbelkörper
- 17: Entlastungszone
- 19: oberer Schaftbereich
- 19a: Gelenkfortsatz
- 21: unterer Schaftbereich
- 21a: Gelenkfortsatz
- 23: Übergangsbereich
- 25: zentrale Bohrung
- 27: Schaftbereich mit reduzierter Querschnittsfläche
- 29: spiralförmige Vertiefung, Schlitz
- 31: Gewinde
- 33: Zwischenstück
- 34: Verankerungsfortsatz
- 35: Gelenk in Entlastungszone
- 37: Gelenk als Entlastungszone
- 38: Gelenkstift
- 39: Querbohrung
- 41: Schlitz
- 43: schlitz-, nut- oder kerbartige Vertiefung
- 45: ebene Stützfläche
- 47: Durchgang
- 49: Gewindeöffnung
- 51: Haltemulde
- 53: Drehpunkt, Togglepoint
- 55: Bandachse
- 57: Schraubenspitze
- 59: Knocheneintritt
- 61: Fixierschraube
- 63: Druck- oder Stützkörper
- 64: Band
- 65: Schlitz
- 66: Vertiefung

- F: intervertebrale Kraft

## Patentansprüche

1. Pedikelschraube, umfassend
einen Kopf (11) zur Koppelung mit einem elastischen intervertebralen Stabilisierungs- oder Stützsystem, wobei der Kopf (11) als eine geschlossene oder einseitig geöffnete Öse ausgebildet ist, die zur Aufnahme eines intervertebralen Stabilisierungs- oder Stützelementes, insbesondere eines Stabes oder Bandes, vorgesehen ist, und einen zur Verankerung in einem Wirbel dienenden Schaft, der sich im implantierten Zustand durch den Pedikel (13) hindurch in den Wirbelkörper (15) erstreckt,
**dadurch gekennzeichnet,**
**dass** der Schaft in seiner vom Kopf (11) aus gemessenen Längserstreckung umfasst:
einen am Kopf (11) angrenzenden oberen Schaftbereich (19) und
eine Entlastungszone (17), die sich an den oberen Schaftbereich (19) anschließt,
wobei die Entlastungszone (17) eine reduzierte Biegesteifigkeit gegenüber dem oberen Schaftbereich (19) aufweist,
wobei die Entlastungszone (17) im zur Anordnung im Knochen vorgesehenen Bereich des Schaftes gelegen ist, und
wobei die Entlastungszone (17) zwischen dem oberen Schaftbereich (19) und einem unteren Schaftbereich (21) gelegen ist, die beide eine höhere Biegesteifigkeit aufweisen als die Entlastungszone (17).

2. Pedikelschraube nach Anspruch 1,
**dadurch gekennzeichnet,**
**dass** der Kopf (11) mit dem oberen Schaftbereich (19) wenigstens in Biegerichtung des Schaftes starr gekoppelt und insbesondere einteilig ist.

3. Pedikelschraube nach Anspruch 1 oder 2,
**dadurch gekennzeichnet,**
**dass** der Gradient der Biegesteifigkeit am Übergang vom oberen Schaftbereich (19) zur Entlastungszone (17) betragsmäßig größer, insbesondere signifikant größer, ist als ein im oberen Schaftbereich (19) auftretender Biegesteifigkeitsgradient.

4. Pedikelschraube nach Anspruch 3,
**dadurch gekennzeichnet,**
**dass** am Übergang vom oberen Schaftbereich (19) zur Entlastungszone (17) wenigstens in einem Bereich des Schaftes der Betrag des Gradienten der Biegesteifigkeit wenigstens doppelt so groß, insbesondere wenigstens 5mal so groß, und weiterhin insbesondere wenigstens 10mal so groß ist wie im oberen Schaftbereich (19).

5. Pedikelschraube nach Anspruch 3 oder 4,
**dadurch gekennzeichnet,**
**dass** die Biegesteifigkeit am Übergang im Wesentlichen sprunghaft reduziert ist.

6. Pedikelschraube nach einem der vorstehenden Ansprüche,
**dadurch gekennzeichnet,**
**dass** die Biegesteifigkeit in der Entlastungszone (17) in wenigstens einer Biegeebene gegenüber der Biegesteifigkeit im oberen Schaftbereich (19) um wenigstens 30%, insbesondere um wenigstens 50%, in einer weiteren Ausführungsform um wenigstens 60%, und in einer noch weiteren Ausführungsform um wenigstens 80% geringer ist.

7. Pedikelschraube nach einem der vorstehenden Ansprüche,
**dadurch gekennzeichnet,**
**dass** der obere Schaftbereich (19) derart dimensioniert ist, dass die Entlastungszone (17) im zur Anordnung in der Spongiosa vorgesehenen Bereich des Schaftes gelegen ist, während wenigstens der zur Anordnung in der Kortikalis vorgesehene Bereich von dem oberen Schaftbereich (19) gebildet ist.

8. Pedikelschraube nach einem der vorstehenden Ansprüche,
**dadurch gekennzeichnet,**
**dass** die Länge des oberen Schaftbereiches (19) im Minimum 5mm, insbesondere im Minimum 8mm sowie im Maximum 15mm, und insbesondere im Maximum 12mm beträgt.

9. Pedikelschraube nach einem der vorstehenden Ansprüche,
**dadurch gekennzeichnet,**
**dass** ein axiales Biegesteifigkeitsprofil des Schaftes wenigstens an den qualitativen Verlauf der Biegesteifigkeit des Pedikels (13) angenähert ist und diesem im Idealfall entspricht.

10. Pedikelschraube nach einem der vorhergehenden Ansprüche,
**dadurch gekennzeichnet,**
**dass** am Übergang von der Entlastungszone (17) zum unteren Schaftbereich (21) wenigstens in einem Bereich des Schaftes der Betrag des Gradienten der Biegesteifigkeit wenigstens doppelt so groß, insbesondere wenigstens 5mal so groß, und weiterhin insbesondere wenigstens 10mal so groß ist wie im unteren Schaftbereich (21).

11. Pedikelschraube nach einem der vorhergehenden Ansprüche,
**dadurch gekennzeichnet,**
**dass** der Wechsel der Biegesteifigkeit von der Entlastungszone (17) zum unteren Schaftbereich (21) im Wesentlichen sprunghaft erfolgt.

12. Pedikelschraube nach einem der vorhergehenden Ansprüche,
**dadurch gekennzeichnet,**
**dass** das Profil des Biegesteifigkeitsverlaufs zwischen dem oberen Schaftbereich (19) und dem unteren Schaftbereich (21) im Wesentlichen topf-, trog- oder wannenförmig ausgebildet ist.

13. Pedikelschraube nach einem der vorstehenden Ansprüche,
**dadurch gekennzeichnet,**
**dass** die Entlastungszone (17) in einem mittleren Bereich der Längserstreckung des Schaftes gelegen ist, insbesondere im Wesentlichen in den beiden mittleren Vierteln des Schaftes oder im mittleren Drittel des Schaftes.

14. Pedikelschraube nach einem der vorstehenden Ansprüche,
**dadurch gekennzeichnet,**
**dass** der obere Schaftbereich (19) und die Entlastungszone (17) derart dimensioniert sind, dass im implantierten Zustand sich die Entlastungszone (17) im Bereich des Übergangs zwischen Pedikel (13) und Wirbelkörper (15) befindet und insbesondere sich auf beiden Seiten axial über den Übergangsbereich (23) hinaus erstreckt, wobei in einer Ausführungsform vom Kopf (11) aus gesehen ein hinter dem Übergang gelegener Abschnitt der Entlastungszone (17) eine größere axiale Länge aufweist als ein vor dem Übergang gelegener Abschnitt der Entlastungszone (17).

15. Pedikelschraube nach einem der vorstehenden Ansprüche,
**dadurch gekennzeichnet,**
**dass** der Schaft mit einem Gewinde (31) versehen ist, wobei das Gewinde (31) durch die Entlastungszone (17) unterbrochen ist.

16. Pedikelschraube nach einem der vorstehenden Ansprüche,
**dadurch gekennzeichnet,**
**dass** der Schaft zumindest bereichsweise hohl ausgeführt und insbesondere mit einer zentralen Längsbohrung (25) versehen ist.

17. Pedikelschraube nach einem der vorstehenden Ansprüche,
**dadurch gekennzeichnet,**
**dass** der Schaft zumindest in der Entlastungszone (17) mit einer Querschnittsschwächung (29, 39, 43, 65) im Vergleich zum oberen Schaftbereich (19) und insbesondere auch zu einem unteren Schaftbereich (21) versehen ist.

18. Pedikelschraube nach einem der vorstehenden Ansprüche,
**dadurch gekennzeichnet,**
**dass** die Entlastungszone (17) von einem lang gestreckten Schaftbereich (27) mit im Vergleich zum oberen Schäftbereich (19) und insbesondere auch zu einem unteren Schaftbereich (21) reduzierter Querschnittsfläche gebildet ist.

19. Pedikelschraube nach einem der vorstehenden Ansprüche,
**dadurch gekennzeichnet,**
**dass** der Schaft zumindest in der Entlastungszone (17) als Wendel ausgebildet ist.

20. Pedikelschraube nach einem der vorstehenden Ansprüche,
**dadurch gekennzeichnet,**
**dass** der Schaft zumindest in der Entlastungszone (17) mit wenigstens einer nut- oder schlitzartigen Vertiefung (29) versehen ist, die insbesondere wendelförmig umläuft.

21. Pedikelschraube nach einem der vorstehenden Ansprüche,
**dadurch gekennzeichnet,**
**dass** die Wandung des Schaftes wenigstens in der Entlastungszone (17) zwei wendelförmig umlaufende schlitzförmige Durchbrechungen aufweist.

22. Pedikelschraube nach Anspruch 21,
**dadurch gekennzeichnet,**
**dass** der Schaft wenigstens im Bereich der Entlastungszone (17) eine Doppelwendel ausbildet.

23. Pedikelschraube nach einem der Ansprüche 19 bis 22,
**dadurch gekennzeichnet,**
**dass** die Steigung der Wendel im Bereich der Entlastungszone (17) wenigstens 5mm, insbesondere wenigstens 10 mm, beträgt.

24. Pedikelschraube nach einem der Ansprüche 19 bis 23,
**dadurch gekennzeichnet,**
**dass** die Steigung der Wendel über die Längserstreckung des Schaftes variiert, wodurch die Biegesteifigkeit des Schaftes über die Längserstreckung variiert.

25. Pedikelschraube nach einem der vorstehenden Ansprüche,
**dadurch gekennzeichnet,**
**dass** der Schaft zumindest in der Entlastungszone (17) mit senkrecht oder schräg zur Schaftachse verlaufenden Querbohrungen (39) versehen ist.

26. Pedikelschraube nach Anspruch 25,
**dadurch gekennzeichnet,**
**dass** von der Außenwand des Schaftes zu den Querbohrungen (39) führende Schlitze (41) angeordnet sind, deren Breite kleiner ist als der Durchmesser der Querbohrungen (39).

27. Pedikelschraube nach einem der Ansprüche 1 bis 18 oder 25 bis 26,
**dadurch gekennzeichnet,**
**dass** der Schaft mit mehreren in axialer Richtung hintereinander und insbesondere versetzt angeordneten, schlitz-, nut- oder kerbartigen Vertiefungen (43) versehen ist, wobei in einer Ausführungsform die Tiefe der Vertiefungen (43) jeweils größer ist als der im Bereich der Vertiefung (43) gemessene halbe Durchmesser des Schaftes.

28. Pedikelschraube nach einem der vorstehenden Ansprüche,
**dadurch gekennzeichnet,**
**dass** das Biegesteifigkeitsprofil des Schaftes bezogen auf dessen Längsachse rotationsunsymmetrisch ist.

29. Pedikelschraube nach einem der vorstehenden Ansprüche,
**dadurch gekennzeichnet,**
**dass** die Entlastungszone (17) derart ausgebildet ist, dass die Biegesteifigkeit in einer Ebene am geringsten ist, die von der Längsachse des Schaftes und der Richtung einer im implantierten Zustand über den Kopf (11) aufgebrachten intervertebralen Kraft aufgespannt ist, wobei in einer Ausführungsform die Biegesteifigkeit in einer senkrecht dazu aufgespannten Ebene am größten ist.

30. Pedikelschraube nach Anspruch 28 oder 29,
**dadurch gekennzeichnet,**
**dass** der Schaft im Bereich der Entlastungszone (17) einen rotationsunsymmetrischen Querschnitt aufweist, und insbesondere in der Ebene der größten Biegesteifigkeit im Wesentlichen eine identische Abmessung aufweist wie die unmittelbar benachbarten oberen und unteren Schaftbereiche (19, 21).

31. Pedikelschraube nach einem der vorstehenden Ansprüche,
**dadurch gekennzeichnet,**
**dass** der Schaft in der Entlastungszone (17) wenigstens einen den Schaft durchdringenden und im Wesentlichen in Längsrichtung des Schaftes verlaufenden Schlitz (65) aufweist.

32. Pedikelschraube nach einem der vorstehenden Ansprüche,
**dadurch gekennzeichnet,**
**dass** der obere Schaftbereich (19) wenigstens eine im Wesentlichen in Längsrichtung verlaufende Vertiefung (66) an der Oberfläche aufweist.

33. Pedikelschraube nach einem der vorstehenden Ansprüche,
**dadurch gekennzeichnet,**
**dass** der Schaft einstückig ausgebildet ist.

34. Pedikelschraube nach einem der Ansprüche 1 bis 32,
**dadurch gekennzeichnet,**
**dass** die Entlastungszone (17) zumindest zum Teil von einem Zwischenstück (33) aus einem vom übrigen Schaftmaterial abweichenden Material gebildet ist.

35. Pedikelschraube nach Anspruch 34,
**dadurch gekennzeichnet,**
**dass** das Zwischenstück (33) aus einem insbesondere faserverstärkten Kunststoffmaterial, insbesondere einem Polymermaterial, hergestellt ist.

36. Pedikelschraube nach einem der Ansprüche 1 bis 32 oder 34 bis 35,
**dadurch gekennzeichnet,**
**dass** an die Entlastungszone (17) angrenzende Schaftbereiche (19, 21) durch ein in der Entlastungszone (17) gelegenes Gelenk (35) miteinander verbunden sind.

37. Pedikelschraube nach einem der Ansprüche 1 bis 32 oder 34 bis 35,
**dadurch gekennzeichnet,**
**dass** die Entlastungszone (17) durch ein Gelenk (37) gebildet ist, das zwei unmittelbar aneinander angrenzende Schaftbereiche (19, 21) miteinander verbindet.

38. Intervertebrales Stabilisierungssystem mit einer Mehrzahl von an den Wirbeln (13, 15) verankerbaren Pedikelschrauben nach einem der vorstehenden Ansprüche und mit einer Verbindungseinrichtung zum Verbinden von zumindest zwei an benachbarten Wirbeln (13, 15) verankerten Pedikelschrauben zu einem elastischen Versteifungs- oder Stützsystem.

39. Intervertebrales Stabilisierungssystem nach Anspruch 38,
**dadurch gekennzeichnet,**
**dass** die Verbindungseinrichtung zur Bildung eines elastischen Stützsystems ein auf Zug vorspannbares Band umfasst, das im implantierten Zustand von wenigstens einem zwischen zwei benachbarten Pedikelschrauben angeordneten kömprimierbaren Druckkörper umgeben ist.

## Claims

1. A pedicle screw, comprising
a head (11) for coupling to an elastic intervertebral stabilization or support system, wherein the head (11) is configured as a closed eye or as an eye that is open at one side, the eye being provided for the reception of an intervertebral stabilization or support element, in particular a rod or a band; and
a shaft which serves for the anchorage in a vertebra and which extends into the vertebral body (15) through the pedicle (13) in the implanted state,
**characterized in that**
the shaft includes in its longitudinal extent measured from the head (11):
an upper shaft region (19) adjacent to the head (11) and
a relief zone (17) adjoining the upper shaft region (19);
with the relief zone (17) having a reduced bending stiffness with respect to the upper shaft region (19);
with the relief zone (17) being disposed in the region of the shaft provided for the arrangement in the bone; and
with the relief zone (17) being disposed between the upper shaft region (19) and a lower shaft region (21) which both have a higher bending stiffness than the relief zone (17).

2. A pedicle screw in accordance with claim 1, **characterized in that** the head (11) is rigidly coupled to, and is in particular in one piece with, the upper shaft region (19) at least in the bending direction of the shaft.

3. A pedicle screw in accordance with claim 1 or 2, **characterized in that** the gradient of the bending stiffness at the transition from the upper shaft region (19) to the relief zone (17) is larger in magnitude, in particular significantly larger, than a bending stiffness gradient occurring in the upper shaft region (19).

4. A pedicle screw in accordance with claim 3, **characterized in that**, at the transition from the upper shaft region (19) to the relief zone (17), the magnitude of the gradient of the bending stiffness is at least twice as large, in particular at least 5 times as large, and furthermore in particular at least 10 times as large in at least one region of the shaft than in the upper shaft region (19).

5. A pedicle screw in accordance with claim 3 or claim 4, **characterized in that** the bending stiffness at the transition is reduced substantially abruptly.

6. A pedicle screw in accordance with any one of the preceding claims, **characterized in that**, in at least one bending plane, the bending stiffness in the relief zone (17) is lower by at least 30%, in particular by at least 50%, in a further embodiment by at least 60%, and in a still further embodiment by at least 80%, with respect to the bending stiffness in the upper region (19).

7. A pedicle screw in accordance with any one of the preceding claims, **characterized in that** the upper shaft region (19) is dimensioned in such a way that the relief zone (17) is disposed in the region provided for arrangement in the spongiosa, whereas at least the region provided for arrangement in the corticalis is formed by the upper shaft region (19).

8. A pedicle screw in accordance with any one of the preceding claims, **characterized in that** the length of the upper shaft region (19) amounts to 5 mm as a minimum, in particular to 8 mm as a minimum, as well as to 15 mm as a maximum and in particular to 12 mm as a maximum.

9. A pedicle screw in accordance with any one of the preceding claims, **characterized in that** an axial bending stiffness profile of the shaft is approximated at least to the qualitative extent of the bending stiffness of the pedicle (13) and, in the ideal case, corresponds to it.

10. A pedicle screw in accordance with any one of the preceding claims, **characterized in that** at the transition from the relief zone (17) to the lower shaft region (21), the magnitude of the gradient of the bending stiffness is at least twice as large, in particular at least five times as large, and furthermore in particular at least 10 times as large in at least one region of the shaft than in the lower shaft region (21).

11. A pedicle screw in accordance with any one of the preceding claims, **characterized in that** the change in the bending stiffness from the relief zone (17) to the lower shaft region (21) takes place substantially abruptly.

12. A pedicle screw in accordance with any one of the preceding claims, **characterized in that** the profile of the bending stiffness development between the upper shaft region (19) and the lower shaft region (21) is made substantially in the form of a pot, a trough or a tub.

13. A pedicle screw in accordance with any one of the preceding claims, **characterized in that** the relief zone (17) is disposed in a central region of the longitudinal extent of the shaft, in particular substantially in the two central quarters of the shaft or in the central third of the shaft.

14. A pedicle screw in accordance with any one of the preceding claims, **characterized in that** the upper shaft region (19) and the relief zone (17) are dimensioned such that, in the implanted state, the relief zone (17) is disposed in the region of the transition between the pedicle (13) and the vertebral body (15) and in particular extends axially beyond the transition region (23) on both sides, with a section of the relief zone (17) disposed behind the transition viewed from the head (11) having a larger axial length in an embodiment than a section of the relief zone (17) disposed in front of the transition.

15. A pedicle screw in accordance with any one of the preceding claims, **characterized in that** the shaft is provided with a thread (31), with the thread (31) being interrupted by the relief zone (17).

16. A pedicle screw in accordance with any one of the preceding claims, **characterized in that** the shaft is hollow at least regionally and is in particular provided with a central bore (25).

17. A pedicle screw in accordance with any one of the preceding claims, **characterized in that**, at least in the relief zone (17), the shaft is provided with a cross-section attenuation (29, 39, 43, 65) in comparison with the upper shaft region (19) and in particular also with a lower shaft region (21).

18. A pedicle screw in accordance with any one of the preceding claims, **characterized in that** the relief zone (17) is formed by an elongated shaft region (27) having a reduced cross-sectional surface in comparison with the upper shaft region (19) and in particular also with a lower shaft region (21).

19. A pedicle screw in accordance with any one of the preceding claims, **characterized in that** the shaft is made as a helix at least in the relief zone (17).

20. A pedicle screw in accordance with any one of the preceding claims, **characterized in that** the shaft is provided with at least one groove-like or slit-like recess (29), at least in the relief zone (17), which in particular extends in a helical shape.

21. A pedicle screw in accordance with any one of the preceding claims, **characterized in that** the wall of the shaft has two slot-like openings having a helical periphery at least in the relief zone (17).

22. A pedicle screw in accordance with claim 21, **characterized in that** the shaft forms a double helix at least in the region of the relief zone (17).

23. A pedicle screw in accordance with any one of the claims 19 to 22, **characterized in that** the pitch of the helix amounts to at least 5 mm, in particular to at least 10 mm, in the region of the relief zone (17).

24. A pedicle screw in accordance with any one of the claims 19 to 23, **characterized in that** the pitch of the helix varies over the longitudinal extent of the shaft, whereby the bending stiffness of the shaft varies over the longitudinal extent.

25. A pedicle screw in accordance with any one of the preceding claims, **characterized in that**, at least in the relief zone (17), the shaft is provided with transverse bores (39) which extend perpendicular or obliquely to the shaft axis.

26. A pedicle screw in accordance with claim 25, **characterized in that** slits (41) are arranged which lead from the outer wall of the shaft to the transverse bores (39) and whose width is smaller than the diameter of the transverse bores (39).

27. A pedicle screw in accordance with any one of claims 1 to 18 or 25 to 26, **characterized in that** the shaft is provided with a plurality of slot-like, groove-like or notch-like recesses (43) arranged in series in the axial direction and in particular offset, with the depth of the recesses (43) in each case being larger than half the diameter of the shaft measured in the region of the recess (43) in an embodiment.

28. A pedicle screw in accordance with any one of the preceding claims, **characterized in that** the bending stiffness profile of the shaft is rotationally asymmetrical with respect to its longitudinal axis.

29. A pedicle screw in accordance with any one of the preceding claims, **characterized in that** the relief zone (17) is configured such that the bending stiffness is lowest in a plane which is spanned from the longitudinal axis of the shaft and the direction of an intervertebral force applied via the head (11) in the implanted state, with the bending stiffness being largest in a plane spanned perpendicular thereto in an embodiment.

30. A pedicle screw in accordance with claim 28 or claim 29, **characterized in that**, in the region of the relief zone (17), the shaft has a rotationally asymmetrical cross-section and in particular substantially has an identical dimension in the plane of the largest bending stiffness to the directly adjacent upper and lower shaft regions (19, 21).

31. A pedicle screw in accordance with any one of the preceding claims, **characterized in that**, in the relief one (17), the shaft has at least one slot (65) passing through the shaft and extending substantially in the longitudinal direction of the shaft.

32. A pedicle screw in accordance with any one of the preceding claims, **characterized in that** the upper shaft region (19) has at least one recess (66) extending substantially in the longitudinal direction at the surface.

33. A pedicle screw in accordance with any one of the preceding claims, **characterized in that** the shaft is made in one piece.

34. A pedicle screw in accordance with any one of the claims 1 to 32, **characterized in that** the relief zone (17) is made at least partly from an intermediate piece (33) which is formed from a material differing from the other shaft material.

35. A pedicle screw in accordance with claim 34, **characterized in that** the intermediate piece (33) is manufactured from a plastic material, in particular a fiber reinforced plastic material, in particular a polymer material.

36. A pedicle screw in accordance with any one of claims 1 to 32 or 34 to 35, **characterized in that** shaft regions (19, 21) adjacent to the relief zone (17) are connected to one another by a joint (35) disposed in the relief zone (17).

37. A pedicle screw in accordance with any one of claims 1 to 32 or 34 to 35, **characterized in that** the relief zone (17) is formed by a joint (37) which connects two shaft regions (19, 21) to one another which are directly adjacent to one another.

38. An intervertebral stabilization system having a plurality of pedicle screws in accordance with any one of the preceding claims which can be anchored to the vertebrae (13), 15) and having a connection device for the connection of at least two pedicle screws anchored to adjacent vertebrae (13, 15) to an elastic stiffening system or support system.

39. An intervertebral stabilization system in accordance with claim 38, **characterized in that** the connection device includes a biased pre-tensionable band for the forming of an elastic support system, the band being surrounded in the implanted state by at least one compressible pressure member arranged between two adjacent pedicle screws.

## Revendications

1. Vis pédiculaire, comportant
une tête (11) destinée à l'accouplement avec un système de stabilisation ou de soutien intervertébral élastique, dans laquelle la tête (11) est réalisée sous la forme d'un oeillet fermé ou ouvert sur un côté qui est prévu pour recevoir un système de stabilisation ou de soutien intervertébral, en particulier un barreau ou un ruban, et une tige qui sert à l'ancrage dans une vertèbre et qui, dans l'état implanté, s'étend à travers le pédicule (13) jusque dans le corps vertébral (15),
**caractérisée en ce que**
la tige comprend, vue dans son extension longitudinale mesurée depuis la tête (11) :
une zone de tige (19) supérieure adjacente à la tête (11) et une zone de décharge (17) qui se raccorde à la zone de tige supérieure (19),
dans laquelle
la zone de décharge (17) présente une rigidité réduite à la flexion par rapport à la zone de tige supérieure (19),
la zone de décharge (17) est située dans la région de la tige prévue pour l'agencement dans l'os, et
la zone de décharge (17) est située entre la zone de tige supérieure (19) et une zone de tige inférieure (21) qui présentent toutes les deux une rigidité à la flexion plus élevée que celle de la zone de décharge (17).

2. Vis pédiculaire selon la revendication 1,
**caractérisée en ce que**
la tête (11) est accouplée rigidement, en particulier d'un seul tenant, à la zone de tige supérieure (19) au moins en direction de flexion de la tige.

3. Vis pédiculaire selon la revendication 1 ou 2,
**caractérisée en ce que**
à la transition de la zone de tige supérieure (19) vers la zone de décharge (17), le gradient de la rigidité à la flexion est d'une valeur supérieure, en particulier significativement supérieure à un gradient de rigidité à la flexion qui se présente dans la zone de tige supérieure (19).

4. Vis pédiculaire selon la revendication 3,
**caractérisée en ce que**
à la transition de la zone de tige supérieure (19) vers la zone de décharge (17), au moins dans une région de la tige, la valeur du gradient de la rigidité à la flexion est au moins deux fois plus élevée, de préférence au moins cinq fois plus élevée, et en outre en particulier au moins dix fois plus élevée que celle dans la zone de tige supérieure (19).

5. Vis pédiculaire selon la revendication 3 ou 4,
**caractérisée en ce que**
au niveau de la transition, la rigidité à la flexion se réduit sensiblement par saut.

6. Vis pédiculaire selon l'une des revendications précédentes,
**caractérisée en ce que**
dans la zone de décharge (17), dans au moins un plan de flexion, la rigidité à la flexion est inférieure d'au moins 30 %, en particulier d'au moins 50 %, selon un autre mode de réalisation d'au moins 60 %, et selon encore un autre mode de réalisation d'au moins 80 % par rapport à la rigidité à la flexion dans la zone de tige supérieure (19).

7. Vis pédiculaire selon l'une des revendications précédentes,
**caractérisée en ce que**
la zone de tige supérieure (19) est dimensionnée de telle sorte que la zone de décharge (19) est située dans la région de la tige prévue pour l'agencement dans le tissu spongieux, tandis qu'au moins la région prévue pour l'agencement de la corticale est formée par la zone de tige supérieure (19).

8. Vis pédiculaire selon l'une des revendications précédentes,
**caractérisée en ce que**
la longueur de la zone de tige supérieure (19) est au minimum de 5 mm, en particulier au minimum de 8 mm, et au maximum de 15 mm, en particulier au maximum de 12 mm.

9. Vis pédiculaire selon l'une des revendications précédentes,
**caractérisée en ce que**
un profil axial de rigidité à la flexion de la tige est au moins approché à l'évolution qualitative de la rigidité à flexion du pédicule (13) et correspond à celle-ci, dans le cas idéal.

10. Vis pédiculaire selon l'une des revendications précédentes,
**caractérisée en ce que**
à la transition de la zone de décharge (17) vers la zone de tige inférieure (21), dans une région au moins de la tige, la valeur du gradient de la rigidité à la flexion est au moins deux fois plus élevée, de préférence au moins cinq fois plus élevée, et en outre en particulier au moins dix fois plus élevée que celle dans la zone de tige inférieure (21).

11. Vis pédiculaire selon l'une des revendications précédentes,
**caractérisée en ce que**
le changement de la rigidité à la flexion depuis la zone de décharge (17) vers la zone de tige inférieure (21) se réduit sensiblement par saut.

12. Vis pédiculaire selon l'une des revendications précédentes,
**caractérisée en ce que**
le profil de l'évolution de la rigidité à la flexion entre la zone de tige supérieure (19) et la zone de tige inférieure (21) est réalisé sensiblement en forme de pot, d'auge ou de cuve.

13. Vis pédiculaire selon l'une des revendications précédentes,
**caractérisée en ce que**
la zone de décharge (17) est située dans une région médiane de l'extension longitudinale de la tige, en particulier sensiblement dans les deux quarts médians de la tige ou dans le tiers médian de la tige.

14. Vis pédiculaire selon l'une des revendications précédentes,
**caractérisée en ce que**
la zone de tige supérieure (19) et la zone de décharge (17) sont dimensionnées de telle sorte que dans l'état implanté, la zone de décharge (17) se trouve au niveau de la transition entre le pédicule (13) et le corps vertébral (15) et s'étend en particulier de part et d'autre axialement au-delà de la zone de transition (23), et dans un mode de réalisation, vu depuis la tête (11), une partie de la zone de décharge (17) située derrière la transition présente une longueur axiale supérieure à celle d'une partie de la zone de décharge (17) située en avant de la transition.

15. Vis pédiculaire selon l'une des revendications précédentes,
**caractérisée en ce que**
la tige est pourvue d'un pas de vis (31), le pas de vis (31) étant interrompu par la zone de décharge (17).

16. Vis pédiculaire selon l'une des revendications précédentes,
**caractérisée en ce que**
la tige est réalisée creuse au moins localement et est en particulier pourvue d'un perçage longitudinal central (25).

17. Vis pédiculaire selon l'une des revendications précédentes,
**caractérisée en ce que**
au moins dans la zone de décharge (17) la tige est pourvue d'un affaiblissement de section transversale (29, 39, 43, 65) par comparaison à la zone de tige supérieure (19) et en particulier également par comparaison à une zone de tige inférieure (21).

18. Vis pédiculaire selon l'une des revendications précédentes,
**caractérisée en ce que**
la zone de décharge (17) est formée par une zone de tige allongée (27) ayant une aire de section réduite par comparaison à la zone de tige supérieure (19) et en particulier également par comparaison à une zone de tige inférieure (21).

19. Vis pédiculaire selon l'une des revendications précédentes,
**caractérisée en ce que**
la tige est réalisée sous forme d'hélice au moins dans la zone de décharge (17).

20. Vis pédiculaire selon l'une des revendications précédentes,
**caractérisée en ce que**
au moins dans la zone de décharge (17), la tige est pourvue d'au moins un renfoncement (29) en forme de gorge ou de fente qui s'étend en particulier en hélice.

21. Vis pédiculaire selon l'une des revendications précédentes,
**caractérisée en ce que**
au moins dans la zone de décharge (17), la paroi de la tige présente deux traversées en forme de fente s'étendant en hélice.

22. Vis pédiculaire selon la revendication 21,
**caractérisée en ce que**
la tige réalise une hélice double au moins dans la région de la zone de décharge (17).

23. Vis pédiculaire selon l'une des revendications 19 à 22,
**caractérisée en ce que**
le pas de l'hélice dans la région de la zone de décharge (17) est d'au moins 5 mm, en particulier d'au moins 10 mm.

24. Vis pédiculaire selon l'une des revendications 19 à 23,
**caractérisée en ce que**
le pas de l'hélice varie sur l'extension longitudinale de la tige, ce pourquoi la rigidité de la tige à la flexion varie sur l'extension longitudinale.

25. Vis pédiculaire selon l'une des revendications précédentes,
**caractérisée en ce que**
au moins dans la zone de décharge (17) la tige est pourvue de perçages transversaux (39) qui s'étendent perpendiculairement ou en oblique par rapport à l'axe de la tige.

26. Vis pédiculaire selon la revendication 25,
**caractérisée en ce que**
il est prévu des fentes (41) qui mènent de la paroi extérieure de la tige jusqu'aux perçages transversaux (39) et dont la largeur est inférieure au diamètre des perçages transversaux (39).

27. Vis pédiculaire selon l'une des revendications 1 à 18 ou 25 à 26,
**caractérisée en ce que**
la tige est pourvue de plusieurs renfoncements (43) en forme de fente, de gorge ou d'encoche, disposés les uns derrière les autres en direction axiale et en particulier en décalage, et selon un mode de réalisation la profondeur respective des renfoncements (43) est supérieure à la moitié du diamètre de la tige mesuré dans la région du renfoncement (43).

28. Vis pédiculaire selon l'une des revendications précédentes,
**caractérisée en ce que**
le profil de la rigidité à la flexion de la tige par rapport à son axe longitudinal n'est pas à symétrie de révolution.

29. Vis pédiculaire selon l'une des revendications précédentes,
**caractérisée en ce que**
la zone de décharge (17) est réalisée de telle sorte que la rigidité à la flexion est la plus faible dans un plan qui est tendu de l'axe longitudinal de la tige et dans la direction d'une force intervertébrale appliquée par la tête (11) dans l'état implanté, et selon un mode de réalisation la rigidité à la flexion est la plus élevée dans un plan tendu perpendiculairement à celui-ci.

30. Vis pédiculaire selon la revendication 28 ou 29,
**caractérisée en ce que**
dans la région de la zone de décharge (17), la tige présente une section qui n'est pas à symétrie de révolution et présente, en particulier dans le plan de la plus grande rigidité à la flexion, une dimension sensiblement identique à celle des zones de tige (19, 21) supérieure et inférieure, directement adjacentes.

31. Vis pédiculaire selon l'une des revendications précédentes,
**caractérisée en ce que**
dans la zone de décharge (17) la tige présente au moins une fente (65) qui traverse la tige et qui s'étend sensiblement en direction longitudinale de la tige.

32. Vis pédiculaire selon l'une des revendications précédentes,
**caractérisée en ce que**
la zone de tige supérieure (19) comprend au moins un renfoncement (66) sur la surface, qui s'étend sensiblement en direction longitudinale.

33. Vis pédiculaire selon l'une des revendications précédentes,
**caractérisée en ce que**
la tige est réalisée d'un seul tenant.

34. Vis pédiculaire selon l'une des revendications 1 à 32,
**caractérisée en ce que**
la zone de décharge (17) est formée au moins en partie par un élément intermédiaire (33) en un matériau qui se distingue du matériau restant de la tige.

35. Vis pédiculaire selon la revendication 34,
**caractérisée en ce que**
l'élément intermédiaire (33) est réalisé en une matière plastique en particulier renforcée par des fibres, en particulier en un matériau de polymère.

36. Vis pédiculaire selon l'une des revendications 1 à 32 ou 34 à 35,
**caractérisée en ce que**
des zones de tige (19, 21) adjacentes à la zone de décharge (17) sont reliées entre elles par une articulation (35) située dans la zone de décharge (17).

37. Vis pédiculaire selon l'une des revendications 1 à 32 ou 34 à 35,
**caractérisée en ce que**
la zone de décharge (17) est formée par une articulation (37) qui relie entre elles deux zones de tige (19, 21) directement adjacentes.

38. Système de stabilisation intervertébral comportant une pluralité de vis pédiculaires selon l'une des revendications précédentes, aptes à être ancrées sur les vertèbres (13, 15), et comportant un dispositif de liaison pour relier au moins deux vis pédiculaires ancrées sur des vertèbres voisines (13, 15) pour former un système élastique de rigidification ou de soutien.

39. Système de stabilisation intervertébral selon la revendication 38,
**caractérisé en ce que**
pour former un système élastique de soutien, le dispositif de liaison comprend un ruban susceptible d'être précontraint en traction qui, dans l'état implanté, est entouré par au moins un corps compressible agencé entre deux vis pédiculaires voisines.
